# EUROPEAN PATENT APPLICATION

(11) **EP 4 556 021 A1**
(43) Date of publication of application: **21.05.2025**
(21) Application number: 23840034.5
(22) Date of filing: 12.07.2023
(51) Int. Cl.: A61K 39/215, A61P 31/14, A61K 39/00

(54) **SARS-COV-2 VACCINE BOOSTER COMPOSITION**

(30) Priority: 12.07.2022 KR 20220085942; 08.08.2022 KR 20220098807; 27.12.2022 KR 20220186401
(71) Applicant: SK Bioscience Co., Ltd., Bundang-gu, Seongnam-si, Gyeonggi-do, 13494 (KR)
(72) Inventor: HONG, Seung Hye, Seongnam-si, Gyeonggi-do 13494 (KR); KANG, Kyung Won, Seongnam-si, Gyeonggi-do 13494 (KR); KIM, Eun Som, Seongnam-si, Gyeonggi-do 13494 (KR); BAIK, Kyung Hwa, Seongnam-si, Gyeonggi-do 13494 (KR); SEOK, Jee Hyun, Seongnam-si, Gyeonggi-do 13494 (KR); SEON, Bo Hyeon, Seongnam-si, Gyeonggi-do 13494 (KR); AHN, Hye Bin, Seongnam-si, Gyeonggi-do 13494 (KR); LEE, Hee Su, Seongnam-si, Gyeonggi-do 13494 (KR); JEONG, Oh Seok, Seongnam-si, Gyeonggi-do 13494 (KR); CHOI, Woo Young, Seongnam-si, Gyeonggi-do 13494 (KR); KIM, Hak, Seongnam-si, Gyeonggi-do 13494 (KR); PARK, Yong Wook, Seongnam-si, Gyeonggi-do 13494 (KR); PARK, Ho Keun, Seongnam-si, Gyeonggi-do 13494 (KR); PAIK, Seung Beom, Seongnam-si, Gyeonggi-do 13494 (KR); SEO, Ki Weon, Seongnam-si, Gyeonggi-do 13494 (KR); YANG, Seon Young, Seongnam-si, Gyeonggi-do 13494 (KR); LEE, Kun Se, Seongnam-si, Gyeonggi-do 13494 (KR); LEE, Su Jeen, Seongnam-si, Gyeonggi-do 13494 (KR)
(74) Representative: Vossius & Partner Patentanwälte Rechtsanwälte mbB
(86) International application number: PCT/KR2023/095028
(87) International publication number: WO 2024/014943

(57) **Abstract**

The present disclosure provides a composition for inducing or maintaining an immune response against SARS-CoV-2 virus.

## Description

### [Technical Field]

The present disclosure relates to a SARS-CoV-2 vaccine booster composition.

### [Background Art]

The SARS-CoV-2 virus, which has been reported to cause infections worldwide since December 2019, is a virus belonging to the *Coronaviridae* family, *Betacoronavirus* genus, *Sarbecovirus* subgenus, and SARS-CoV-2 virus infection is also referred to as COVID-19 (coronavirus disease 2019). The main route of transmission includes close contact with droplets of an infected person, and it is known that the virus can also be transmitted by directly contacting an infected person, or by touching an intermediary carrier such as an object contaminated with droplets of an infected person and then touching the eyes, nose, or mouth without washing hands.

It is known that SARS-CoV-2 causes infection when a receptor binding domain (RBD) at the top of trimer glycoproteins (i.e., spike proteins) on the surface of the virus binds to an ACE2 receptor protein on the surface of human cells. The spike glycoprotein monomer is cleaved into the S1 subunit and S2 subunit by host cell proteases.

### [Prior Art Documents]

### [Patent Document]

(Patent Document 1) US 9630994 B2

### [Non-Pent Document]

(Non-Pent Document 1) Science. 2016 Jul 22;353(6297):389-94.

### [Disclosure]

### [Technical Problem]

The global pandemic of the SARS-CoV-2 virus has resulted in an increase in deaths and massive socioeconomic damage, and as the damage has been rapidly increasing, rapid vaccine development is required, and vaccine compositions for preventing SARS-CoV-2 infection or alleviating symptoms thereof are being developed.

### [Technical Solution]

It is one object of the present disclosure to provide a booster vaccine composition for inducing or maintaining an immune response against a wild-type and variant SARS-CoV-2 in an administered subject, wherein the subject has been vaccinated at least once with a vaccine composition against SARS-CoV-2 or has a history of infection with SARS-CoV-2.

It is another object of the present disclosure to provide a SARS-CoV-2 vaccine composition for use in a method for inducing or maintaining an immune response against a wild-type and variant SARS-CoV-2 in an administered subject, including: a first step of administering a vaccine composition against SARS-CoV-2; and a second step of administering a vaccine composition against SARS-CoV-2 at least 14 days after the first step.

It is still another object of the present disclosure to provide the use of a composition for inducing or maintaining an immune response against a wild-type and variant SARS-CoV-2 viruses.

It is yet another object of the present disclosure to provide a method for inducing or maintaining an immune response against a wild-type and variant SARS-CoV-2 viruses.

### [Advantageous Effects]

The composition of the present disclosure maintains IgG antibodies and neutralizing antibodies at high levels without side effects, thereby forming or maintaining immunity against SARS-CoV-2 virus infection at high levels. Additionally, it can form immunity against variant SARS-CoV-2 infections, thereby enabling effective prevention against various variant SARS-CoV-2 virus infections that may occur in evolutionary patterns.

### [Brief Description of Drawings]

FIG. 1 shows the effect of booster shots on the formation of immunity against the SARS-CoV-2 Wuhan strain, as confirmed by FRNT50 IU/mL.
FIG. 2 shows the effect of booster shots on the formation of immunity against the SARS-CoV-2 Wuhan strain, as confirmed by FRNT50 in the original scale.
FIG. 3 shows the effect of booster shots on the formation of immunity against the SARS-CoV-2 Wuhan strain, as confirmed by ELISA IgG Ab titer, BAU/mL.
FIG. 4 shows the effect of booster shots on the formation of immunity against the SARS-CoV-2 Wuhan strain, as confirmed by ICS Assay. ICS Assay was performed on only 17 out of 81 participants.
FIG. 5 shows the effect of booster shots on the formation of immunity against the SARS-CoV-2 Omicron variant, as confirmed by FRNT50 in the original scale.
FIG. 6 shows the results of confirming adverse events (AE) when booster shots were administered compared to when only the prime vaccine was administered.
FIG. 7 shows the results of an immunogenicity test in which serum samples were analyzed at 2, 5, and 8 weeks after vaccinating mice three times with the vaccine as described in Example 3.
FIG. 8 shows the results of a CPE-based neutralization test using serum samples collected at 8 weeks after the first immunization in mice as described in Example 3.
FIG. 9 to 11 show the results of a challenge test performed after vaccinating mice three times as described in Example 3, and recording the body weight, body temperature, and survival rate.
FIG. 12 shows the results of a live virus challenge test in mice, showing the level of virus load in the lungs.
FIG. 13 shows the results of observing the virus load (top), cytokine analysis (middle), and lung histopathology (bottom) in mice.
FIG. 14 shows the results of confirming the titer of RBD-specific IgG in a primate model.
FIGS. 15 and 16 show the results of PBNA measurement for wild-type and variant SARS-CoV-2 in a primate model. FIG. 15 shows the results for wild-type, Delta, and Beta variants, and FIG. 16 shows the results for the Omicron variant and wild-type SARS-CoV-2.
FIG. 17 shows the results of confirming IFN-gamma secreting T cells that specifically react to the RBD antigen in a primate model.
FIGS. 18 and 19 show the results of confirming the viral load measured using nasal swabs (FIG. 18) and bronchoalveolar lavage (FIG. 19) in a primate model, respectively.
FIG. 20 shows the results of confirming cross-immunity formation for Omicron subvariants (BA.1, BA.5, XBB.1, BN.1) through reduction neutralizing antibody titration (FRNT).

### [Detailed Description of Preferred Embodiments]

One aspect of the present disclosure provides a booster vaccine composition for inducing or maintaining an immune response against SARS-CoV-2 in an administered subject.

In one embodiment, the booster vaccine composition includes: (i) a receptor binding domain (RBD) nanoparticle, which includes (a) a trimer consisting of three assembled first polypeptide monomers including an amino acid sequence of SEQ ID NO: 1 or an amino acid sequence having at least 75% identity thereto; and (b) a pentamer consisting of five assembled second polypeptide monomers including an amino acid sequence of SEQ ID NO: 2 or an amino acid sequence having at least 75% identity thereto, and (ii) AS03.

In another embodiment, the subject has been vaccinated at least once with a vaccine composition against SARS-CoV-2 or has a history of infection with SARS-CoV-2.

In still another embodiment, the subject has been vaccinated at least twice with a vaccine composition against SARS-CoV-2 or has a history of infection with SARS-CoV-2 after being vaccinated with a vaccine composition.

As the booster vaccine composition according to any one of the embodiments described above, the first polypeptide monomer includes an RBD sequence of a wild-type or variant SARS-CoV-2 virus.

As the booster vaccine composition according to any one of the embodiments described above, the first polypeptide monomer includes an RBD sequence of a wild-type or Beta variant SARS-CoV-2 virus.

As the booster vaccine composition according to any one of the embodiments described above, the first polypeptide monomer includes an RBD sequence selected from SEQ ID NO: 21 and SEQ ID NO: 22.

As the booster vaccine composition according to any one of the embodiments described above, the first polypeptide monomer includes an RBD sequence of a wild-type SARS-CoV-2 virus.

As the booster vaccine composition according to any one of the embodiments described above, the first polypeptide monomer includes an RBD sequence of SEQ ID NO: 21.

The booster vaccine composition according to any one of the embodiments described above is a composition, in which a first composition, which includes an RBD nanoparticle consisting of a first polypeptide monomer including an RBD sequence of SEQ ID NO: 21; and a second polypeptide monomer including an amino acid sequence of SEQ ID NO: 2, and AS03; and
a second composition, which includes an RBD nanoparticle consisting of a first polypeptide monomer including an RBD sequence of SEQ ID NO: 22; and a second polypeptide monomer including an amino acid sequence of SEQ ID NO: 2, and AS03, are mixed.

The booster vaccine composition according to any one of the embodiments described above induces an immune response against SARS-CoV-2 virus including a wild-type and variants.

The booster vaccine composition according to any one of the embodiments described above induces an immune response against SARS-CoV-2 virus including the Alpha, Beta, Delta, Gamma, and Omicron variants.

The booster vaccine composition according to any one of the embodiments described above induces an immune response against SARS-CoV-2 virus selected from at least one of the Alpha, Beta, Delta, Gamma, and Omicron variants.

As the booster vaccine composition according to any one of the embodiments described above, the vaccine composition administered to a subject for administration includes: (i) a receptor binding domain (RBD) nanoparticle, including (a) a trimer consisting of three assembled first polypeptide monomers including an amino acid sequence of SEQ ID NO: 1 or an amino acid sequence having at least 75% identity thereto; and (b) a pentamer consisting of five assembled second polypeptide monomers comprising an amino acid sequence of SEQ ID NO: 2 or an amino acid sequence having at least 75% identity thereto, and (ii) AS03.

As the booster vaccine composition according to any one of the embodiments described above, the subject is primed with a vaccine different from the booster vaccine composition.

As the booster vaccine composition according to any one of the embodiments described above, the subject is primed with the same vaccine as the booster vaccine composition.

As the booster vaccine composition according to any one of the embodiments described above, the vaccine composition ("prime vaccine composition") administered to a subject for administration includes a viral vector, nucleic acid or synthetic antigen as an active ingredient.

As the booster vaccine composition according to any one of the embodiments described above, the first polypeptide included in the vaccine composition administered to a subject for administration includes an RBD sequence of a wild-type SARS-CoV-2 virus.

As the booster vaccine composition according to any one of the embodiments described above, the first polypeptide included in the vaccine composition administered to a subject for administration includes an RBD sequence of SEQ ID NO: 21.

As the booster vaccine composition according to any one of the embodiments described above, the first polypeptide included in the vaccine composition administered to a subject for administration consists of an amino acid sequence of SEQ ID NO: 1.

As the booster vaccine composition according to any one of the embodiments described above, the second polypeptide included in the vaccine composition administered to a subject for administration consists of an amino acid sequence of SEQ ID NO: 2.

Another aspect of the present disclosure provides a SARS-CoV-2 vaccine composition for use in a method for inducing or maintaining an immune response against SARS-CoV-2 in an administered subject.

In one embodiment, the method includes: a first step of administering a prime vaccine composition against SARS-CoV-2, thereby forming immunity; and a second step of administering a booster vaccine composition against SARS-CoV-2 at least 14 days after the first step.

In another embodiment, the SARS-CoV-2 vaccine composition of the first and/or the second step includes (i) a receptor binding domain (RBD) nanoparticle, including (a) a trimer consisting of three assembled first polypeptide monomers including an amino acid sequence of SEQ ID NO: 1 or an amino acid sequence having at least 75% identity thereto; and (b) a pentamer consisting of five assembled second polypeptide monomers including an amino acid sequence of SEQ ID NO: 2 or an amino acid sequence having at least 75% identity thereto, and (ii) AS03.

As the composition according to any one of the embodiments described above, the interval between the first and second steps is 28 days or more.

As the composition according to any one of the embodiments described above, the vaccine composition administered in the first step includes a viral vector, nucleic acid or synthetic antigen as an active ingredient.

As the composition according to any one of the embodiments described above, the prime vaccine composition administered in the first step is administered once or twice.

As the composition according to any one of the embodiments described above, the prime vaccine composition administered in the first step is administered twice at a time interval of 28 to 300 days.

As the composition according to any one of the embodiments described above, the interval between the first and second steps is at least 28 days.

As the composition according to any one of the embodiments described above, the second step is performed 28 to 300 days after the first step.

As the composition according to any one of the embodiments described above, the first polypeptide monomer includes an RBD sequence of a wild-type or Beta variant.

As the composition according to any one of the embodiments described above, the first polypeptide monomer includes an RBD sequence selected from SEQ ID NO: 21 and SEQ ID NO: 22.

As the composition according to any one of the embodiments described above, the first polypeptide monomer included in the SARS-CoV-2 vaccine composition administered in the second step includes an RBD sequence of a wild-type or variant SARS-CoV-2 virus.

As the composition according to any one of the embodiments described above, the first polypeptide monomer included in the SARS-CoV-2 vaccine composition administered in the second step includes an RBD sequence of a wild-type or Beta SARS-CoV-2 virus.

As the composition according to any one of the embodiments described above, the first polypeptide monomer included in the SARS-CoV-2 vaccine composition administered in the second step includes an RBD sequence of a wild-type or Beta SARS-CoV-2 virus.

As the composition according to any one of the embodiments described above, the SARS-CoV-2 vaccine composition administered in the second step includes an RBD sequence selected from SEQ ID NO: 21 and SEQ ID NO: 22.

As the composition according to any one of the embodiments described above, the SARS-CoV-2 vaccine composition administered in the second step includes an RBD sequence of a wild-type SARS-CoV-2 virus.

As the composition according to any one of the embodiments described above, the SARS-CoV-2 vaccine composition administered in the second step includes an RBD sequence of a wild-type SARS-CoV-2 virus.

As the composition according to any one of the embodiments described above, the SARS-CoV-2 vaccine composition administered in the second step includes an RBD sequence of SEQ ID NO: 21.

As the composition according to any one of the embodiments described above, the SARS-CoV-2 vaccine composition administered in the second step includes an RBD sequence is a composition, in which a first composition, which includes an RBD nanoparticle consisting of a first polypeptide monomer including an RBD sequence of SEQ ID NO: 21; and a second polypeptide monomer including an amino acid sequence of SEQ ID NO: 2, and AS03; and
a second composition, which includes an RBD nanoparticle consisting of a first polypeptide monomer including an RBD sequence of SEQ ID NO: 22; and a second polypeptide monomer including an amino acid sequence of SEQ ID NO: 2, and AS03, are mixed

As the composition according to any one of the embodiments described above, the first polypeptide included in the vaccine composition administered in the first and/or the second step includes an RBD sequence of SEQ ID NO: 21.

As the composition according to any one of the embodiments described above, the first polypeptide included in the vaccine composition administered in the first and/or the second step includes an RBD sequence of a wild-type SARS-CoV-2 virus.

As the composition according to any one of the embodiments described above, the first polypeptide included in the vaccine composition administered in the first and/or the second step includes an RBD sequence of SEQ ID NO: 21.

As the composition according to any one of the embodiments described above, the first polypeptide included in the vaccine composition administered in the first and/or the second step consists of an amino acid sequence of SEQ ID NO: 1.

As the composition according to any one of the embodiments described above, the second polypeptide included in the vaccine composition administered in the first and/or the second step consists of an amino acid sequence of SEQ ID NO: 2.

The composition according to any one of the embodiments described above is used in a method for inducing or maintaining an immune response against a wild-type and/or variant SARS-CoV-2 viruses.

The composition according to any one of the embodiments described above is used in a method for inducing or maintaining an immune response against SARS-CoV-2 virus including Alpha, Beta, Delta, Gamma, and Omicron variants.

The composition according to any one of the embodiments described above is used in a method for inducing or maintaining an immune response against SARS-CoV-2 virus of any one or more of the Alpha, Beta, Delta, Gamma, and Omicron variants.

The composition according to any one of the embodiments described above includes (i) RBD nanoparticles and (ii) AS03 in a ratio of about 1:1.

The composition according to any one of the embodiments described above includes RBD nanoparticles in an amount of 5 µg to 25 µg in a single dose.

The composition according to any one of the embodiments described above is administered by intramuscular injection.

The composition according to any one of the embodiments described above is provided in the form of a pharmaceutical package or kit.

The composition according to any one of the embodiments described above includes a means for administering the composition to a patient.

Still another aspect of the present disclosure provides the use of a composition for inducing or maintaining an immune response against SARS-CoV-2, wherein the composition includes (i) a receptor binding domain (RBD) nanoparticle, including (a) a trimer consisting of three assembled first polypeptide monomers comprising an amino acid sequence of SEQ ID NO: 1 or an amino acid sequence having at least 75% identity thereto; and (b) a pentamer consisting of five assembled second polypeptide monomers comprising an amino acid sequence of SEQ ID NO: 2 or an amino acid sequence having at least 75% identity thereto, and (ii) AS03.

In one embodiment, the composition is used for the purpose of inducing or maintaining an immune response against a wild type and variant SARS-CoV-2.

As the use according to any one of the embodiments described above, the composition is used for the purpose of inducing or maintaining an immune response against SARS-CoV-2 formed by a prime vaccine composition.

As the use according to any one of the embodiments described above, the prime vaccine composition includes a viral vector, nucleic acid or synthetic antigen as an active ingredient.

Yet another aspect of the present disclosure provides a method for inducing or maintaining an immune response against the SARS-CoV-2 virus.

In one embodiment, the method is a method for inducing or maintaining an immune response against a wild-type and variant SARS-CoV-2 viruses.

As the method according to any one of the embodiments described above, the method includes a step of administering a composition, which includes: (i) a receptor binding domain (RBD) nanoparticle, including (a) a trimer consisting of three assembled first polypeptide monomers including an amino acid sequence of SEQ ID NO: 1 or an amino acid sequence having at least 75% identity thereto; and (b) a pentamer consisting of five assembled second polypeptide monomers including an amino acid sequence of SEQ ID NO: 2 or an amino acid sequence having at least 75% identity thereto, and (ii) AS03, to a subject.

As the method according to any one of the embodiments described above, the method is used for the purpose of inducing or maintaining an immune response against SARS-CoV-2 formed by a prime vaccine composition.

The present disclosure will be described in detail as follows. Meanwhile, each description and embodiment disclosed herein can be applied to other descriptions and embodiments, respectively. That is, all combinations of various elements disclosed herein fall within the scope of the present disclosure. Further, the scope of the present disclosure is not limited by the specific description described below.

Additionally, those of ordinary skill in the art may be able to recognize or confirm, using only conventional experimentation, many equivalents to the particular aspects of the disclosure described herein. Furthermore, it is also intended that these equivalents be included in the present disclosure.

Additionally, a number of papers and patent documents have been cited throughout the present specification, and their citations are indicated. The content of the cited papers and patent documents is incorporated herein by reference in their entirety, and the level of technical field to which the present disclosure belongs and the contents of the present disclosure will be described more clearly.

As used herein, the term "about" may be presented before a particular numerical value. The term "about" used herein includes not only the exact number recited after the term, but also a range that is near or close to that number. Considering the context in which the number is presented, it can be determined whether any number is close to or near the particular number presented. In one example, the term "about" may refer to a range from -10% to +10% of a numerical value. In another example, the term "about" may refer to a range from -5% to +5% of a given numerical value, but is not limited thereto.

The term "and/or" where used herein is to be taken as specific disclosure of each of the two specified features or components with or without the other. Thus, the term "and/or" as used in a phrase such as "A and/or B" herein is intended to include "A and B," "A or B," "A" (alone), and "B" (alone). Likewise, the term "and/or" as used in a phrase such as "A, B, and/or C" is intended to encompass each of the following aspects: A, B, and C; A, B, or C; A or C; A or B; B or C; A and C; A and B; B and C; A (alone); B (alone); and C (alone).

As used herein, the term "consisting essentially of" may mean that, in the case that the features of the object claimed herein are not substantially affected by the presence of an unspecified component, the unspecified component may be present.

As used herein, the term, "consisting of" means that the total ratio of specific component(s) is 100%. The components or features that are recited after the term "consisting of" may be essential or mandatory. In some embodiments, in addition to the components or features recited after the term "consisting of", any other components or non-essential components may be excluded.

As used herein, the term "comprising/including" means the presence of features, steps or components recited after the term, and does not exclude the presence or addition of one or more features, steps or components. The components or features recited after the term "comprising/including" herein may be essential or mandatory. However, in some embodiments, the term may further include any other or non-essential components or features.

As used herein, the term "comprising/including" may be modified to refer to "consisting essentially of" or "consisting of" in some embodiments.

With respect to amino acid sequences in the present disclosure, although it is described as a polypeptide "comprising/including" an amino acid sequence described by a specific sequence number, a polypeptide "consisting of" an amino acid sequence described by a specific sequence number, or a polypeptide or protein "having" an amino acid sequence described by a specific sequence number, it is apparent that any protein having an amino acid sequence in which part of the sequence is deleted, modified, substituted, conservatively substituted or added can be used in the present disclosure if it has the same or corresponding activity as the polypeptide consisting of the amino acid sequence of the corresponding sequence number. For example, it may be a case in which the N-terminus and/or C-terminus of the amino acid sequence is added with a sequence that does not alter the function of the protein, a naturally occurring mutation, a silent mutation thereof, or a conservative substitution, but is not limited thereto.

### RBD Nanoparticles and Immunoadjuvants

The RBD nanoparticle of the present disclosure is a multimeric protein assembly, and it may also be referred to as a "nanostructure", "RBD nanostructure", or "RBD-NP", and may include (i) a trimer consisting of three assembled first polypeptide monomers including an amino acid sequence of SEQ ID NO: 1 or an amino acid sequence having at least 75% identity thereto; and (ii) a pentamer consisting of five assembled second polypeptide monomers including an amino acid sequence of SEQ ID NO: 2 or an amino acid sequence having at least 75% identity thereto.

The first polypeptide may include a receptor binding domain (RBD) for SARS-CoV-2 spike glycoproteins; a first self-assembling polypeptide that acts as a structure of the nanoparticles; and a linker connecting the RBD and the first self-assembling polypeptide.

In one example, the RBD sequence may be an RBD sequence of a wild-type SARS-CoV-2 virus. In another example, the RBD sequence may be an RBD sequence of a variant SARS-CoV-2 virus. Examples of the sequence of the variant SARS-CoV-2 virus include an RBD sequence of the virus selected from Beta, Delta, and Omicron, as shown in SEQ ID NOS: 34 to 36. One example of the variant SARS-CoV-2 virus may include a Beta variant.

In one example, the RBD sequence may be represented by an amino acid sequence selected from SEQ ID NOS: 21 to 24.

In one example, the RBD sequence may be represented by an amino acid sequence selected from SEQ ID NO: 21 or SEQ ID NO: 22.

In one example, the RBD sequence may be a sequence represented by SEQ ID NO: 21.

The first polypeptide monomer may further include an N-terminal phosphatase signal peptide sequence (MGILPSPGMPALLSLVSLLSVLLMGCVA). The several isolated first polypeptide monomers can self-assemble through interaction to form a trimer. Such an assembly can be made through a non-covalent protein-protein interaction reaction.

The second polypeptide is a second self-assembling polypeptide monomer that serves as a structure of the nanoparticles, and the several isolated second polypeptide monomers can self-assemble through interaction to form a pentamer. Such an assembly can be made through a non-covalent protein-protein interaction reaction.

In the present disclosure, the first polypeptide may include one of the polypeptides described in International Patent Publication No. 2019-169120 as the first self-assembling polypeptide. Specifically, the first polypeptide monomer may be a polypeptide consisting of an amino acid sequence of SEQ ID NO: 1 or an amino acid sequence having at least 75% identity thereto.

In one example, the first polypeptide may be a polypeptide consisting of an amino acid sequence represented by SEQ ID NO: 1.

In another example, the first polypeptide may be a polypeptide consisting of an amino acid sequence in which an amino acid sequence corresponding to the RBD of SARS-CoV-2 in the amino acid sequence represented by SEQ ID NO: 1 is substituted with a Beta variant of the SARS-CoV-2 RBD sequence.

In the present specification, the polypeptide consisting of the amino acid sequence represented by SEQ ID NO: 1, which is an example of the first polypeptide, may be referred to as "RBD-Component A", "Component A", or "RBD-153-50A".

Additionally, the polypeptide consisting of an amino acid sequence represented by SEQ ID NO: 5, which is an example of the first self-assembling polypeptide included in the polypeptide of SEQ ID NO: 1, may be referred to as "153-50A".

In one example, the first polypeptide may be encoded by a gene consisting of a nucleic acid sequence represented by SEQ ID NO: 3. In another example, the first polypeptide may be a polypeptide in which the region corresponding to the sequence encoding the RBD of SARS-CoV-2 in the nucleic acid sequence represented by SEQ ID NO: 3 is substituted with a RBD sequence of a Beta variant of SARS-CoV-2. One example of the RBD sequence of the Beta variant SARS-CoV-2 may be a sequence of SEQ ID NO: 22.

The second polypeptide monomer may be a polypeptide consisting of an amino acid sequence of SEQ ID NO: 2 or an amino acid sequence having at least 75% identity thereto, and, for example, may be a polypeptide consisting of an amino acid sequence represented by SEQ ID NO: 2.

In the present specification, the polypeptide of SEQ ID NO: 2 may be referred to as "153-50B" or "Component B". The second polypeptide monomer may be encoded by a gene consisting of a nucleic acid sequence represented by SEQ ID NO: 4.

The first polypeptide and the second polypeptide may include any modification that does not disrupt assembly as a multimeric protein assembly, and, for example, may include deletion, insertion, substitution, or modification of an amino acid.

The first polypeptide and the second polypeptide may include proteins disclosed in International Patent Publication No. 2019-169120, U.S. Patent No. 9630994, International Patent Publication Nos. 2021-163481 and 2021-163438, or International Application No. PCT/US2021/037341.

In one example, the first self-assembling polypeptide included in the first polypeptide, which is a polypeptide consisting of the amino acid sequence of SEQ ID NO: 1 or an amino acid sequence having at least 85% identity thereto, may be a polypeptide consisting of any one of the amino acid sequences of SEQ ID NOS: 6 to 10 (corresponding to the amino acid sequence of SEQ ID NOS: 7, 29, 30, 31, or 39 of International Patent Publication No. 2019-169120).

In one example, the second polypeptide, which is a polypeptide consisting of the amino acid sequence of SEQ ID NO: 2 or an amino acid sequence having at least 85% identity thereto, may be a polypeptide consisting of any one of the amino acid sequences of SEQ ID NOS: 9 to 16 (corresponding to the amino acid sequence of SEQ ID NOS: 6, 8, 10, 27, 28, 32, 33, or 38 of International Patent Publication No. 2019-169120).

The RBD nanoparticles may be formed by self-assembly of a trimer of the first polypeptide and a pentamer of the second polypeptide. Specifically, the RBD nanoparticles of the present disclosure may have a structure including 20 trimers of the (i) first polypeptide (Component A) and 12 pentamers of the (ii) second polypeptide (Component B). Accordingly, the multimeric protein assembly of the present disclosure includes 60 copies of the first polypeptide (Component A) and 60 copies of the second polypeptide (Component B).

The RBD nanoparticles of the present disclosure may have an icosahedral symmetrical structure. The RBD nanoparticles of the present disclosure may have a diameter of about 30 nm to about 70 nm, and in particular, the lumen may be about 15 nm to about 32 nm in length. For example, they may have a diameter of about 28 nm excluding the RBD antigen region.

The cell line producing the first polypeptide monomer may be a cell line having an expression vector including a gene encoding a protein represented by the amino acid sequence of SEQ ID NO: 1. In one specific embodiment of the present disclosure, the expression vector may be a plasmid vector, but any vector suitable for expressing the first polypeptide monomer in the cell line may be used without limitation.

The cell line producing the second polypeptide monomer may be a cell line having an expression vector including a gene encoding a protein represented by the amino acid sequence of SEQ ID NO: 2. In one specific embodiment of the present disclosure, the expression vector may be a pET-based vector, for example, a pET29b+ vector, but any vector suitable for expressing the second polypeptide monomer in the cell line may be used without limitation.

In one specific embodiment of the present disclosure, an expression vector including a gene encoding a protein represented by the amino acid sequence of SEQ ID NO: 1 herein may have a DNA sequence represented by SEQ ID NO: 19.

In one specific embodiment of the present disclosure, an expression vector including a gene encoding a protein represented by the amino acid sequence of SEQ ID NO: 2 herein may have a DNA sequence represented by SEQ ID NO: 20.

It is understood that the amino acid sequences or nucleic acid sequences used herein include sequences that show substantial identity to the sequences described in the sequence list. Herein, the "substantial identity" means any sequence different from the sequences of the present disclosure, and may be a sequence having at least 75%, 80%, 85%, 90%, 91%, 92%, 93%, 94%, 95%, 96%, 97%, 98%, 99% or more homology thereto, and it is understood that the amino acid sequences or nucleic sequences within the homology range are also included in the scope of the present disclosure.

As used herein, the term "vector" refers to a nucleic acid including a nucleotide sequence that can be introduced into a host cell to be recombined and inserted into the genome of the host cell, or can be spontaneously replicated as an episome. Suitable expression vectors include expression regulatory elements, such as promoters, start codons, stop codons, polyadenylation signals, and enhancers, as well as signal sequences or leader sequences for membrane targeting or secretion, and may be prepared variously according to their purpose. The start codon and stop codon must act in an individual when a gene construct encoding a target protein is administered thereto, and must be in-frame in the coding sequence.

The polynucleotide or vector according to one embodiment of the present disclosure may exist in a genetically modified host cell or non-human host subject as an independent molecule, specifically as a replicable molecule, outside the genome thereof, or may be stably inserted into the genome of the host cell or non-human host subject.

The host cell of the cell line producing the first polypeptide monomer according to one embodiment of the present disclosure is a eukaryotic cell. The eukaryotic cell includes a fungal cell, a plant cell, or an animal cell. Examples of the fungal cell may include yeast, specifically, yeast of *Saccharomyces* sp., and more specifically, *Saccharomyces cerevisiae.* Additionally, examples of the animal cell include insect cells or mammalian cells, and specific examples of the animal cell may include HEK293, 293T, NSO, Chinese hamster ovary cells (CHO), MDCK, U2-OSHela, NIH3T3, MOLT-4, Jurkat, PC-12, PC-3, IMR, NT2N, Sk-n-sh, CaSki, C33A, *etc.* In addition, suitable cell lines well known in the art may be obtained from cell line depositories such as American Type Culture Collection (ATCC).

The host cell of the cell line producing the first polypeptide monomer according to one specific embodiment of the present disclosure may be a Chinese hamster ovary cell (CHO), specifically, HD-BIOP3 cells in which exon 6 of the glutamine synthetase gene is knocked out by recombinant adeno-associated virus (rAAV) may be used.

In order to introduce an expression vector having a genetic sequence encoding the first polypeptide monomer into the host cell, any method for introducing a nucleic acid into a cell may be used, and techniques known in the art may be used depending on the host cell. For example, the method may include heat shock, electroporation, CaPO₄ precipitation, CaCl₂ precipitation, microinjection, a polyethylene glycol (PEG) method, a DEAE-dextran method, a cationic liposome method, or a lithium acetate-DMSO method, *etc.,* but is not limited thereto.

The vaccine composition of the present disclosure may include a pharmaceutically acceptable carrier together with the RBD nanoparticles. As used herein, the term "pharmaceutically acceptable carrier" includes any and all solvents, dispersion media, coatings, surfactants, antioxidants, preservatives (e.g., antibacterial agents, antifungal agents), isotonic agents, absorption delaying agents, salts, preservatives, drug stabilizers, binders, excipients, disintegration agents, lubricants, sweetening agents, flavoring agents, dyes, *etc.,* and combinations thereof, as known to those skilled in the art in the technical field to which the present disclosure pertains. Except insofar as any conventional carrier is incompatible with the active ingredient, its use in the therapeutic or pharmaceutical compositions is contemplated.

In one specific embodiment of the present disclosure, the vaccine composition of the present disclosure may consist of a finished drug product and an immunoadjuvant. The finished drug product may contain a buffer, a stabilizer, and a solvent in addition to the RBD nanoparticles, as the main component. Sodium chloride and tromethamine may be used as the buffer, and L-arginine and white sugar (sucrose) may be used as the stabilizer. In addition, a composition including a combination of an adjuvant, a stabilizer, an isotonic agent, and a buffer may be used as the immunoadjuvant.

As used herein, the term "immunoadjuvant" refers to a substance that non-specifically stimulates an immune response to an antigen in the initial process of activating immune cells, and the vaccine composition of the present disclosure includes various immunoadjuvants that can enhance an immune response when administered together with RBD nanoparticles, and the immunoadjuvant of the present disclosure may be AS03. The AS03 of the present disclosure may include polysorbate-80, squalene, and alpha-tocopherol.

In the present disclosure, the immunoadjuvant may include an adjuvant, a stabilizer, an isotonic agent, and a buffer.

The adjuvant may include both squalene and alpha-tocopherol. As used herein, the term "adjuvant" means to include an oil-in-water (W/O) emulsion. The squalene may include squalene, an analogue thereof, or any pharmaceutically acceptable oil. The alpha-tocopherol may include alpha-tocopherol or an analogue, and a derivative thereof.

The squalene (2,6,10,15,19,23-Hexamethyl-2,6,10,14,18,22-tetracosahexaene) is an unsaturated oil which is found in large quantities in shark-liver oil, and in lower quantities in olive oil, wheat germ oil, rice bran oil, and yeast.

The squalene may be contained in an amount of about 5 mg to 15 mg, may be contained in an amount of 6 mg to 14 mg, 7 mg to 13 mg, 8 mg to 12 mg, 9 mg to 11 mg, and 10 mg to 11 mg, or may be contained in an amount of about 10 mg to 11 mg, per 0.25 ml of an immunoadjuvant. If the squalene is contained in an amount less than 5 mg, it may be difficult to induce sufficient immunogenicity, and if it is contained in an amount exceeding 15 mg, there is a possibility that the disturbance of lipid metabolism of cells increases. If squalene is contained in an immunoadjuvant within the above range, the immunogenicity of RBD nanoparticles used as an antigen may be enhanced (Example 1).

The alpha-tocopherol corresponds to a sterol, and other sterols applicable to the present disclosure include β-sitosterol, stigmasterol, ergosterol, and ergocalciferol.

The alpha-tocopherol may be contained in an amount of about 5 mg to 15 mg, or may be contained in an amount of 6 mg to 14 mg, 7 mg to 13 mg, 8 mg to 12 mg, 9 mg to 12 mg, or 10 mg to 12 mg, or may be contained in an amount of 10 mg to 12 mg, per 0.25 ml of an immunoadjuvant. When the alpha-tocopherol is contained in an immunoadjuvant within the above range, the immunogenicity of RBD nanoparticles used as an antigen may be enhanced (Example 1).

The stabilizer may include polysorbate 80. As used herein, the "stabilizer" means to include a surfactant. Other surfactants applicable to the present disclosure may be polysorbate 20, polysorbate 21, polysorbate 40, polysorbate 60, polysorbate 61, polysorbate 65, polysorbate 81, polysorbate 85, PEG3350, and mixtures thereof.

The polysorbate 80 may be contained in an amount of about 1 mg to 10 mg, or may be contained in an amount of about 2 mg to 9 mg, 3 mg to 8 mg, 4 mg to 7 mg, 4 mg to 6 mg, or 4 mg to 5 mg, or may be contained in an amount of 4 mg to 5 mg, per 0.25 ml of an immunoadjuvant. When the polysorbate 80 is contained in an immunoadjuvant within the above range, the immunogenicity of RBD nanoparticles used as an antigen may be enhanced (Example 1).

The isotonic agent may include both sodium chloride and potassium chloride. As used herein, the "isotonic agent" refers to an agent that plays the role of maintaining a suitable osmotic pressure *in vivo* when a vaccine is administered in a liquid form into the body.

The sodium chloride may be contained in an amount of 100 mM to 150 mM, or may be contained in an amount of 103 mM to 147 mM, 106 mM to 144 mM, 109 mM to 141 mM, 112 mM to 138 mM, 115 mM to 135 mM, 118 mM to 132 mM, 120 mM to 129 mM, 120 mM to 126 mM, or 120 mM to 123 mM, or may be contained in an amount of 120 mM to 123 mM, per 0.25 ml of an immunoadjuvant. When the sodium chloride is contained in an immunoadjuvant within the above range, the immunogenicity of RBD nanoparticles used as an antigen may be enhanced (Example 1).

The potassium chloride may be contained in an amount of about 0.1 mM to 10 mM, or may be contained in an amount of 0.5 mM to 9 mM, 1 mM to 8 mM, 1.5 mM to 7 mM, 2 mM to 6 mM, 2 mM to 5 mM, 2 mM to 4 mM, or 2 mM to 3 mM, or may be contained in an amount of 2 mM to 3 mM, per 0.25 ml of an immunoadjuvant. When the potassium chloride is contained in an immunoadjuvant within the above range, the immunogenicity of RBD nanoparticles used as an antigen may be enhanced (Example 1).

The buffer may include both sodium phosphate and potassium phosphate. As used herein, the term "buffer" refers to an agent that plays the role of maintaining the pH of a solution so that the pH does not change rapidly within the solution to which it is added.

The sodium phosphate may be contained in an amount of about 4 mM to 12 mM, or may be contained in an amount of 4.5 mM to 11.5 mM, 5 mM to 11 mM, 5.5 mM to 10.5 mM, 6 mM to 10 mM, 7 mM to 9.5 mM, 7 mM to 9 mM, 7 mM to 8.5 mM, or 7 mM to 8 mM, or may be contained in an amount of 7 mM to 8 mM, per 0.25 ml of an immunoadjuvant. When the sodium phosphate is contained in an immunoadjuvant within the above range, the immunogenicity of RBD nanoparticles used as an antigen may be enhanced (Example 1).

The potassium phosphate may be contained in an amount of about 0.01 mM to 5 mM, or may be contained in an amount of 0.05 mM to 4.5 mM, 0.1 mM to 4 mM, 0.5 mM to 3.5 mM, 1 mM to 3 mM, or 1 mM to 2 mM, or may be contained in an amount of 1 mM to 2 mM, per 0.25 ml of an immunoadjuvant. When the potassium phosphate is contained in an immunoadjuvant within the above range, the immunogenicity of RBD nanoparticles used as an antigen may be enhanced (Example 1).

The immunoadjuvant may include squalene and alpha-tocopherol as adjuvants, polysorbate 80 as a stabilizer, sodium chloride and potassium chloride as isotonic agents, and sodium phosphate and potassium phosphate as buffers. Alternatively, an immune response against SARS-CoV-2 may be induced when a combination of two or more components used in the immunoadjuvant is formulated together with the RBD nanoparticles and administered to a subject. When the immunoadjuvant is mixed with the RBD nanoparticles, which are antigens, and administered, the IgG antibody titer and the neutralizing antibody titer increase, thereby obtaining an excellent prevention effect of SARS-CoV-2 infection (Example 1).

In one example, the immunoadjuvant and the RBD nanoparticles may be each stored in separate containers. In one embodiment of the present disclosure, the immunoadjuvant; and a finished drug product containing the RBD nanoparticles, sodium chloride, tromethamine, and L-arginine and sucrose may be provided in a form in which each is stored in a separate container, and may be mixed immediately before vaccination to be used.

Since AS03 exists in the form of an emulsion, it is be provided in a separate container, thus reducing the possibility of changes in physical properties due to the vaccine and the mixed solution, and helping to secure the stability of the antigen material of the vaccine.

In another example, the immunoadjuvant may be mixed with the finished drug product and stored.

The immunoadjuvant may be mixed in a dose of 0.5 to 3 times per single dose of the RBD nanoparticles, or may be mixed in a dose of 0.6 to 2.9 times, 0.7 to 2.8 times, 0.8 to 2.7 times, 0.9 to 2.6 times, 1.0 to 2.5 times, 1.1 to 2.4 times, 1.2 to 2.3 times, 1.3 to 2.2 times, 1.4 to 2.1 times, 1.5 to 2.0 times, 1.6 to 1.9 times, or 1.7 to 1.8 times, or may be mixed in a dose of 0.7 to 1.5 times or 1.0 to 1.2 times, or may be mixed in a ratio of 1:1, but is not limited thereto. If the immunoadjuvant is contained within the above range, the IgG antibody titer and neutralizing antibody titer increase, thereby obtaining an excellent prevention effect of SARS-CoV-2 infection (Example 1).

The composition of the present disclosure may be used to form or maintain immunity against the SARS-CoV-2 virus.

As used herein, the term "SARS-CoV-2 (SARS-coronavirus-2)" refers to a virus that causes coronavirus disease-19 (COVID-19), a respiratory disease. Thus, the immunogenic composition of the present disclosure or a vaccine composition or booster composition including the same may be used to prevent coronavirus disease-19 (COVID-19).

The SARS-CoV-2 virus of the present disclosure may also include, without limitation, all variant forms that can be classified as SARS-CoV-2, and may include mutations of the following: examples include Alpha (e.g., B.1.1.7), Beta (e.g., B.1.351), Gamma (e.g., P.1, P.1.1, P.1.2), Delta (e.g., B.1.617.2), Epsilon (e.g., B.1.427, B.1.429), Eta (e.g., B.1.525), Iota (e.g., B.1.526), Kappa (e.g., B.1.617.1), Zeta (e.g., P.2), Mu (e.g., B.1.621, B.1.621.1), Omicron (e.g., B.1.1.529), BA lineages (e.g., BA.1, BA.5, *etc.),* XBB lineages (XBB.1, *etc*.), BN lineages (BN.1 *etc*.), BQ lineages, *etc.* In one example, the SARS-CoV-2 virus of the present disclosure may include one or more selected from Alpha, Beta, Gamma, Delta, and Omicron variant viruses. Additionally, the virus is not limited to the names of the variants (Alpha, Beta, Gamma, Delta, and Omicron), and may also include sub-lineages reclassified from the variant viruses without limitation.

In the present disclosure, the wild-type SARS-CoV-2 virus may also be referred to as the Wuhan strain.

Information related to the nucleic acid sequences of the SARS-CoV-2 virus and the amino acid sequences of the protein of the present disclosure can be obtained from a known database, such as NCBI Genbank, GISAID, or by analyzing the viruses provided by the National Culture Collections for Pathogens under the Korea Disease Control and Prevention Agency. For example, information on the wild-type Wuhan strain (Wuhan/WIV04/2019, BetaCoV/Korea/KCDC03/2020) can be obtained from GISAID accession ID: EPI_ISL_402124, information on the Beta variant of the SARS-CoV-2 virus can be obtained from GISAID accession ID: EPI_ISL_712096, and information on the Delta variant of the SARS-CoV-2 virus can be obtained from GenBank Accession ID: QTW89558.1.

As used herein, the term "vaccine" means a composition including at least one immunologically active component that induces an immunological response in an animal. For the purpose of the present disclosure, the immunologically active component may be RBD nanoparticles.

As used herein, the term "booster vaccine composition" means a composition including a certain dose of an immunogen that is administered to a patient to enhance, prolong, or maintain protective immunity. The booster vaccine composition may be administered to overcome the downregulation of IgG antibody titer and neutralizing antibody titer.

The subject to whom the vaccine composition or the booster vaccine composition of the present disclosure is administered may be any mammal, specifically humans.

The subject to whom the booster vaccine composition of the present disclosure is administered may be an individual who has already formed the protective immunity through exposure to an immunogenic substance through previous vaccination ("primed individual"). In this case, the vaccine composition which is administered at an interval from the time of previous vaccination may be referred to as a "booster vaccine composition", and the previous vaccine may be referred to as a "prime vaccine".

In one example, the protective immunity may have been formed by the prime vaccine. The prime vaccine may be a vaccine different from the booster vaccine composition or may be the same vaccine. The prime vaccine composition may be administered an appropriate number of times, for example, once or twice, to form protective immunity, and when administered more than twice, the vaccine composition may be administered at an appropriate time interval.

In one example, the subject to whom the booster vaccine composition of the present disclosure is administered, may have been previously vaccinated at least once, or may have been vaccinated twice

In one example, the vaccine previously administered to the subject to whom the booster vaccine composition of the present disclosure is administered ("prime vaccine"), may be a vaccine containing a viral vector (e.g., ChAdOx1 nCOV-19 from AstraZeneca, Ad26.COV2.S from Janssen), a nucleic acid such as mRNA (BNT162b2 from Pfizer, mRNA-1273 from Moderna), or a synthetic antigen (NVX-CoV2373 from Novavax, Skycovione from SK Bioscience) as an active ingredient.

In one example, the vaccine previously administered to the subject receiving the booster vaccine composition may be a composition including the **RBD** nanoparticles described above as an immunologically active component.

Administration of the composition of the present disclosure may be made by infusion or injection (e.g., intravenously, intramuscularly, intradermally, subcutaneously, intrathecal, intraduodenally, intraperitoneally, *etc.).* **The** vaccine composition may also be administered intranasally, vaginally, rectally, orally, or transdermally. Alternatively, the vaccine composition may be administered by "needle-free" delivery systems. **In one** example, the composition of the present disclosure may be administered by intramuscular injection.

When the composition of the present disclosure is administered more than twice, the time interval between the administrations of the composition may be, for example, 14 days or more.

In one example, the booster vaccine composition of the present disclosure may be administered 28 days or more after the last vaccination. For example, the booster vaccine composition of the present disclosure may be administered 28 to 300 days after the last vaccination.

In one example, if the subject has not previously been exposed to an immunogenic substance, the vaccine composition may be administered three or more times. Regarding the above administration, the time interval between the first administration and the second administration may be 14 days or more, for example, 28 days or more, *e.g.,* 28 to 32 days. Regarding the above administration, the time interval between the second administration and the third administration may be 14 days or more, for example, 28 days or more, *e.g.,* 28 to 300 days. In another example, the time interval may be 12 weeks or more. In one example, the booster vaccine composition of the present disclosure may be administered 28 days or more after the last vaccination of the prime vaccine. In one example, the booster vaccine composition of the present disclosure may be administered 12 weeks or more after the last vaccination of the prime vaccine.

Since the composition of the present disclosure also has an effect of forming a protective immunity against a variant SARS-CoV-2 virus to which the subject has not been previously exposed, the composition of the present disclosure can be administered to induce an immune response against the SARS-CoV-2 virus in the subject.

The dose of the composition of the present disclosure may be dependent on many variables, including the species, breed, size, height, weight, age, overall health of a patient, the type of formulation, the mode or manner of administration, *etc.* In one example, the composition of the present disclosure may contain RBD nanoparticles in an amount of about 5 µg or 25 µg per dose. In one example, the composition of the present disclosure may contain RBD nanoparticles in an amount of about 10 µg or 25 µg per dose. In one example, the composition of the present disclosure may contain SARS-CoV-2 RBD nanoparticles in an amount of about 5 µg, 10 µg or 25 µg per 0.25 ml. In particular, the AS03 to be administered may be administered in the same volume as the composition including the RBD nanoparticles. In one example, the composition of the present disclosure may be administered together with a 0.25 ml of receptor binding domain (RBD) nanoparticle composition containing SARS-CoV-2 RBD nanoparticles in an amount of 5 µg, 10 µg or 25 µg, and 0.25 ml of AS03.

As used herein, the term "prevention" refers to any activity that inhibits or delays the onset of a disease.

The composition of the present disclosure may be provided in the form of a pharmaceutical package or kit.

It is also envisaged that the pharmaceutical package or kit of the present disclosure further includes means for administration to a patient and/or buffers, vials, teflon bags, or infusion bags that are normally used for the infusion of immunogenic compositions. The "means" thereby includes one or more articles selected from the group consisting of a syringe, a hypodermic needle, a cannula, a catheter, an infusion bag, vehicles, vials, buffers, stabilizers, and written instructions to aid the skilled person in the preparation of the respective doses and infusion bags of the disclosure, *etc.*

### [Mode for Carrying Out the Invention]

Hereinafter, the present disclosure will be described in detail by way of Examples and Experimental Examples. However, these Examples and Experimental Examples are merely preferred Examples given for illustrative purposes, and thus, the scope of the present disclosure is not intended to be limited to or by these Examples and Experimental Examples.

### Preparation Example 1: Preparation of RBD Nanoparticles

The trimer glycoproteins on the surface of the SARS-CoV-2 virus are also referred to as spike glycoproteins, and the receptor binding domain (RBD) at the top of the trimer glycoproteins binds to the ACE2 receptor protein on the surface of human cells, thereby causing infection. The RBD is combined with nanoparticles to produce RBD-nanoparticles.

The RBD-nanoparticles are formed by self-assembly of 20 assembled trimers of Component A having an amino acid sequence represented by SEQ ID NO: 1 and 12 pentamers of Component B having an amino acid sequence represented by SEQ ID NO: 2. The RBD-Component A is composed of the RBD of the SARS-CoV-2 spike glycoproteinss, I53-50A, which serves as a structure of the nanoparticles, and a linker (GGSGGSGSGGSGGSGSEKAAKAEEAAR) connecting the RBD and I53-50A.

In order to prepare a recombinant nanoparticle coronavirus vaccine, a CHO cell line (stable cell line) was used to prepare the RBD-Component A protein, and an E. *coli* cell line was used to prepare the Component B protein. The preparation of each component is described in detail in Preparation Examples 1-1 and 1-2 below.

### 1-1. Preparation of RBD-Component A Protein

For the preparation of RBD-Component A, which is used as an antigen for the SARS-CoV-2 vaccine, the HD-BIOP3 cell line provided by HORIZON Discovery, UK, was used as a host cell. Additionally, the RBD Component A cDNA (NT-SARS-CoV-2RBD-I53-50A-16GS-he), which was synthesized using the Sall/Notl restriction enzyme reaction, was inserted into the donor plasmid (pJV145) to produce a recombinant expression vector plasmid (M-2560). The M-2560 vector has a DNA sequence represented by SEQ ID NO: 19. Information on the location of major genes and functions in the recombinant expression vector plasmid (M-2560) is summarized in Table 1.

**[Table 1]**

| Location of Genes in Expression Vector | Information |
|---|---|
| Nucleotide position 440-593 | SV40 early promoter |
| Nucleotide position 668-1,789 | GS gene |
| Nucleotide position 2,257-2,319 | 3' PiggyBac transposon-specific right ITR(inverted terminal repeat sequence) |
| Nucleotide position 2,951-2,985 | 5' PiggyBac transposon-specific left ITR |
| Nucleotide position 3,275-4,477 | cHS4 insulator gene |
| Nucleotide position 4,484-4,955 | mCMV promoter |
| Nucleotide position 6,264-7,667 | NT-SARS-CoV-2RBD-153-50A-16GS-he |

A CHO cell line-derived Component A intermediate crude solution was obtained by conventional protein expression and purification methods. As a result of peptide mapping analysis of the CHO cell line-derived Component A intermediate crude solution, 100% peptide coverage was obtained, which was confirmed to be mutually equivalent to the predicted peptide sequence, thereby confirming that the intermediate crude solution was the target Component A protein.

### 1-2. Preparation of Component B Protein

For the preparation of Component B protein, the E. *coli* BL21 strain provided by Thermofisher was used. pET29b+ was used as a vector for the expression of Component B protein. When the gene of Component B protein was inserted into pET29b+, an I53-50B vector with a total sequence of 5.7 kb (vector: 5.4 kb) was produced. The vector consists of a kanamycin resistance gene, a Lac operon whose transcription is induced by lactose and its derivatives, a T7 promoter region derived from lambda phage, a multiple cloning site, *etc.* The multiple cloning site has several restriction enzyme sequences and is a site that plays a major role in the introduction of antigen proteins. The I53-50B vector has a DNA sequence represented by SEQ ID NO: 20. The main genetic information in the I53-50B vector is shown in Table 2 below.

**[Table 2]**

| | |
|---|---|
| | |
| Nucleotide position 12-467 | f1 origin |
| Nucleotide position 560-1375 | KanR |
| Nucleotide position 1471-2144 | pUCorigin |
| Nucleotide position 4598-4675 | LacI promoter |
| Nucleotide position 4988-5007 | T7promotor |
| Nucleotide position 5007-5031 | Lac operator |
| Nucleotide position 5076-5549 | I53-50B |
| Nucleotide position 5645-5688 | T7terminator |

The *E. coli-*derived Component B intermediate crude solution was obtained by conventional protein expression and purification methods. The amino acid sequences of the Component B intermediate crude solution were confirmed to be 100% identical to the theoretical amino acid sequences.

### 1-3. Preparation of Recombinant RBD Nanoparticle Crude Solution

The prepared Component A intermediate crude solution and Component B intermediate crude solution were used to prepare a recombinant RBD nanoparticle crude solution (raw drug product) through an assembly process.

### 1-3-1. Preparation of Recombinant RBD Nanoparticle Crude Solution

The Component A crude solution and Component B crude solution were mixed and assembled so that the reaction ratio was 1:1.1 in molar concentration. Herein, the reaction conditions were as follows: the reaction was carried out at room temperature for 1 hour with a stirring speed of 80 rpm, and when the reaction was completed, the reaction solution was filtered through a 0.2 µm filter. The thus-filtered assembly reaction solution was concentrated to 4 kg using a tangential flow filtration system equipped with a 300 kDa molecular weight cut-off (MWCO) membrane with an area of 0.7 m² (ultrafiltration), and the solution was recovered by buffer exchange using a 50 mM Tris buffer solution having a volume 10 times that of the concentrate (diafiltration). Herein, the transmembrane pressure (TMP) was maintained at 1 bar or below. The ultrafiltration recovery solution was filtered through a 0.2 µm filter and stored in an ultra-low temperature freezer at -70°C or below.

### 1-3-2. Confirmation of Particle Morphology of Recombinant RBD Nanoparticle Crude Solution

The recombinant RBD nanoparticle crude solution is a particle-shaped molecule formed by the combination of the Component A intermediate crude solution and the Component B intermediate crude solution. These particle-shaped molecules are designed computationally so that molecules of a certain size are formed when an appropriate combination occurs. Among the methods for measuring the molecular size of proteins, the commonly used method is the dynamic light scattering method. The size of the nanoparticle molecules formed through the above can be measured by dynamic light scattering, which is commonly used method, thus, it is possible to confirm whether an appropriate structure has been formed. The size of the nanoparticle crude solution prepared through the assembly process using the Component A intermediate crude solution and the Component B intermediate crude solution was measured by dynamic light scattering, and as a result, it was confirmed that the nanoparticle crude solution had a size of 40 to 70 nm, which conformed to the theoretically predicted value.

### 1-4. Preparation of RBD Nanoparticle AS03 Formulation

The final crude solution was prepared through a formulation process. In the formulation process, AS03 (GSK), a composition including an adjuvant, a stabilizer, an isotonic agent, and a buffer, was used as an immunoadjuvant. Aluminum hydroxide was mixed into the final solution in advance. The final solution was named RBD nanoparticle AS03 formulation.

### Example 1. Confirmation of Immunogenicity of RBD Nanoparticle AS03 Formulation

The experiment was conducted on 80 adults aged 19 to 85 years. The study intervention is defined as any investigational intervention, product, placebo, or medical device that is considered to be imparted to study participants according to the study protocol. Table 3 shows the dose and specification of the raw drug product (finished drug product) (0.25 ml) used to mix AS03 (GSK) as an immunoadjuvant, Table 4 shows the dose and specification of the raw drug product (0.5 ml), in which AS03 is mixed (hereinafter referred to as RBD nanoparticle AS03 formulation), and Table 5 shows the dose and specification of the raw drug product (0.5 ml), in which AS03 is not mixed. Table 6 shows the information on the raw drug products involved.

**[Table 3]**

| Dose and Specification of Raw Drug Product used to Mix AS03 (0.25 ml) | | | |
|---|---|---|---|
| | Raw Material | High dose (25 µg/dose¹⁾) | Low dose (10 µg/dose¹⁾) |
| Main ingredient | Recombinant COVID-19 surface antigen protein nanoparticles (RBD nanoparticles of Preparation Example 1) | 25 µg¹⁾ | 10 µg¹⁾ |
| Buffer | Sodium chloride | 2.192 mg | 2.192 mg |
| Buffer | Tromethamine | 1.514 mg | 1.514 mg |
| Stabilizer | L-arginine | 4.355 mg | 4.355 mg |
| Stabilizer | White sugar | 12.5 mg | 12.5 mg |
| Solvent | Sterile water for injection | Appropriate amount | Appropriate amount |

| | | | |
|---|---|---|---|
| ¹⁾ Dose as recombinant COVID-19 surface antigen protein (RBD) | | | |

**[Table 4]**

| Dose and Specification of Raw Drug Product in which AS03 is mixed (0.5 ml) | | | |
|---|---|---|---|
| RBD nanoparticle 0.25 ml | | | |
| | Raw Material | High dose (25 µg/dose¹⁾) | Low dose (10 µg/dose¹⁾) |
| Main ingredient | Recombinant COVID-19 surface antigen protein nanoparticles (RBD nanoparticles of Preparation Example 1) | 25 µg¹⁾ | 10 µg¹⁾ |
| Buffer | Sodium chloride | 2.192 mg | 2.192 mg |
| Buffer | Tromethamine | 1.514 mg | 1.514 mg |
| Stabilizer | L-arginine | 4.355 mg | 4.355 mg |
| Stabilizer | White sugar | 12.5 mg | 12.5 mg |
| Solvent | Sterile water for injection | Appropriate amount | Appropriate amount |

| Immunoadjuvant AS03 0.25 ml | | | |
|---|---|---|---|
| Adjuvant | Squalene | 10.69 mg | 10.69 mg |
| Stabilizer | Alpha-tocopherol | 11.86 mg | 11.86 mg |
| Stabilizer | Polysorbate 80 | 4.86 mg | 4.86 mg |
| Isotonic agent | Sodium chloride | 121 mM | 121 mM |
| Isotonic agent | Potassium chloride | 2.38 mM | 2.38 mM |
| Buffer | Sodium phosphate | 7.14 mM | 7.14 mM |
| Buffer | Potassium phosphate | 1.30 mM | 1.30 mM |
| Solvent | Sterile water for injection | Appropriate amount | Appropriate amount |

| | | | |
|---|---|---|---|
| ¹⁾ Dose as recombinant COVID-19 surface antigen protein (RBD) | | | |

**[Table 5]**

| Dose and Specification of Raw Drug Product in which AS03 is not mixed (0.5 ml | | | |
|---|---|---|---|
| | Raw Material | High dose (25 µg/dose¹⁾) | Low dose (10 µg/dose¹⁾) |
| Main ingredient | Recombinant COVID-19 surface antigen protein nanoparticles | 25 µg¹⁾ | 10 µg¹⁾ |
| Buffer | Sodium chloride | 4.383 mg | 4.383 mg |
| Buffer | Tromethamine | 3.029 mg | 3.029 mg |
| Stabilizer | L-arginine | 8.71 mg | 8.71 mg |
| Stabilizer | White sugar | 25 mg | 25 mg |
| Solvent | Sterile water for injection | Appropriate amount | Appropriate amount |

| | | | |
|---|---|---|---|
| ¹⁾ Dose as recombinant COVID-19 surface antigen protein (RBD) | | | |

**[Table 6]**

| Invention Name | SARS-CoV-2 recombinant protein nanoparticle vaccine (RBD nanoparticle)-AS03 |
|---|---|
| Type | Vaccine |
| Dose formulation | RBD, self-assembling protein nanoparticles, exposed on the outer surface of SARS-CoV-2 |
| Single dose | RBD nanoparticles (RBD 10 µg or 25 µg/dose) adjuvanted with/without AS03; total injection volume is 0.5 mL. |
| Dosage level | RBD nanoparticles (single administration): 10 µg or 25 µg of SARS-CoV-2 RBD nanoparticles per 0.5 mL (AS03 mixed administration): 10 µg or 25 µg of SARS-CoV-2 RBD nanoparticles per 0.25 ml, AS03: 0.25 ml. |
| Mode of administration | Intramuscular injection |
| Storage condition | 2°C to 8°C |

For the vaccination study, a saline placebo (0.5 ml), RBD nanoparticles, in which AS03 is mixed, or RBD nanoparticles, in which AS03 is not mixed, were used. Herein, the RBD content in RBD nanoparticles was 10 ug or 25 ug. The placebo, RBD nanoparticle-AS03 formulation, and RBD nanoparticles were each injected intramuscularly twice. In order to aid the RBD nanoparticle vaccine with AS03 in a 1:1 ratio, 0.35 ml of AS03 was withdrawn from a vial containing approximately 3 ml of AS03 and added to another vial containing 0.35 ml of RBD nanoparticles (RBD 10 ug or 25 ug per dose). In each administration, 0.5 ml was withdrawn from any one of a vial containing 0.65 ml of RBD nanoparticles alone, a vial containing 0.7 ml of RBD nanoparticles, in which AS03 is mixed, or a 20 ml of saline ampoule. The remaining volume in the vial or ampoule was discarded. In the vaccine study, injections were administered into the deltoid muscle of the upper arm on days 0 and 28 with a 28-day interval.

Table 7 shows the experimental subjects of the AS03 formulation. The intention-to-treat set (ITT set), safety set, full analysis set (FA set), and per protocol set (PP set) in Table 7 are analysis populations widely known in pharmaceutical clinical trials. The analysis population refers to the group of subjects included in the statistical analysis among the subjects who participated in the clinical trial.

**[Table 7]**

| | RBD nanopartic le 10 ug AS03 formulation | RBD nanopart icle 10 ug | RBD nanoparti cle 25 ug AS03 formulation | RBD nanoparti cle 25 ug | Placeb o | Total |
|---|---|---|---|---|---|---|
| Random arrangement, n | 20 | 10 | 20 | 10 | 20 | 80 |
| ITT set, n (%) | 20 (100.00) | 10 (100.00) | 20 (100.00) | 10 (100.00) | 20 (100.0 0) | 80 (100.00) |
| Safety set, n (%) | 20 (100.00) | 10 (100.00) | 20 (100.00) | 10 (100.00) | 20 (100.0 0) | 80 (100.00) |
| FA set, n (%) | 20 (100.00) | 10 (100.00) | 20 (100.00) | 10 (100.00) | 20 (100.0 0) | 80 (100.00) |
| Exclusion criteria from PP, n (%) | 0 (0.00) | 0 (0.00) | 2 (10.00) | 1 (10.00) | 1 (5.00) | 4 (5.00) |
| Participants did not complete vaccination schedule. | 0 (0.00) | 0 (0.00) | 0 (0.00) | 1 (10.00) | 0 (0.00) | 1 (1.25) |
| Participants did not receive study intervention at appropriate time. | 0 (0.00) | 0 (0.00) | 1 (5.00) | 0 (0.00) | 0 (0.00) | 1 (1.25) |
| Any of the blood samples were not collected or were not collected at appropriate time by Visit 7 (28 days + 5 after Visit 4)) | 0 (0.00) | 0 (0.00) | 1 (5.00) | 0 (0.00) | 1 (5.00) | 2 (2.50) |
| PP set, n (%) | 20 (100.00) | 10 (100.00) | 18 (90.00) | 9 (90.00) | 19 (95.00) | 76 (95.00) |
| ITT set (%): (Number of ITT set / number of participants in random arrangement) * 100 | | | | | | |
| Safety set (%): (Number of safety set / number of ITT set) * 100 | | | | | | |
| FA set (%): (number of FA set / number of safety set) * 100 | | | | | | |
| Exclusion criteria from PP set (%): (Number of exclusion criteria PP set / number of FA set) * 100 | | | | | | |
| PP set (%): (number of PP set / number of FA set) * 100 | | | | | | |

### 1-1. Confirmation of Immunogenicity by Geometric Mean Titer and Seroconversion Rate of IgG Antibodies

The RBD nanoparticle AS03 formulation (10 µg or 25 µg) and RBD nanoparticles alone (10 µg or 25 µg) were administered to the experimental subjects described in Table 7 above, and the geometric mean titer and seroconversion rate of IgG antibodies against SARS-CoV-2-RBD were confirmed by ELISA. The geometric mean titer (GMT) is a method of calculating the average antibody titer for a group of test subjects by multiplying all values and taking the n^{th} root of the values (where n is the number of test subjects with available data). A geometric mean fold rise (GMFR) represents the increase rate of the geometric mean concentration or geometric mean titer after vaccination relative to the baseline before vaccination. A confidence interval (CI) of 95% is observed, and the confidence interval provides a range of values that can be the difference between the population mean and the target value. Table 8 shows the geometric mean titer and seroconversion rate of IgG antibodies for the administration of RBD nanoparticle AS03 formulations (low and high doses) and RBD nanoparticles alone, and the "t" in the results of "P-value vs. placebo" in Table 8 represents the "t-test" result. The t-test is a statistical hypothesis test that used to compare means.

**[Table 8]**

| | RBD nanoparticle 10 ug AS03 formulation (N=20) | RBD nanoparticl e 10 ug(N=10) | RBD nanoparticle 25 ug AS03 formulation (N=18) | RBD nanoparticl e 25 ug(N=9) | Placebo (N=19) |
|---|---|---|---|---|---|
| Geometric Mean Titer (GMT) and Seroconversion Rate Baseline | | | | | |
| n | 20 | 10 | 18 | 9 | 19 |
| GMT±standard deviation | 11.81±1.56 | 10.95±1.61 | 12.34±1.62 | 13.10±1.46 | 10.74±1. 61 |
| 95% CI (lower limit, upper limit) | (9.58, 14.55) | (7.78, 15.40) | (9.71, 15.67) | (9.77, 17.57) | (8.54, 13.49) |
| P-vale vs. placebo | 0.5218 t | 0.9175 t | 0.3823 t | 0.2821 t | |
| P-value vs. RBD nanoparticle 10 ug AS03 formulation | | 0.6709 t | 0.7732 t | | |
| P-vale vs. RBD nanoparticle 25 ug AS03 formulation | | | | 0.7478 t | |

| Visit 4 (4 weeks after first vaccination) | | | | | |
|---|---|---|---|---|---|
| n | 20 | 10 | 18 | 9 | 19 |
| GMT±standard deviation | 192.85±1.88 | 37.72±2.03 | 212.06±2.10 | 33.57±1.70 | 13.58±1. 64 |
| 95% CI (lower limit, upper limit) | (143.40, 259.35) | (22.74, 62.56) | (146.66, 306.62) | (22.28, 50.59) | (10.68, 17.26) |
| P-value vs. placebo | <0.0001 t | 0.0001 t | <0.0001 t | 0.0002 t | |
| P-value vs. RBD nanoparticle 10 ug AS03 formulation | | <0.0001 t | 0.6728 t | | |
| P-value vs. RBD nanoparticle 25 ug AS03 formulation | | | | <0.0001 t | |
| | | | | | |
| GMFR±standard deviation | 16.33±2.31 | 3.45±1.91 | 17.19±2.41 | 2.56±1.77 | 1.26±1.5 9 |
| 95% CI (lower limit, upper limit) | (11.03, 24.17) | (2.17, 5.47) | (11.10, 26.62) | (1.65, 3.98) | (1.01, 1.58) |
| P-value vs. placebo | <0.0001 t | <0.0001 t | <0.0001 t | 0.0018 t | |
| P-value vs. RBD nanoparticle 10 ug AS03 formulation | | <0.0001 t | 0.8552 t | | |
| P-value vs. RBD nanoparticle 25 ug AS03 formulation | | | | <0.0001 t | |
| | | | | | |

| Visit 6 (2 weeks after second vaccination) | | | | | |
|---|---|---|---|---|---|
| n | 20 | 10 | 18 | 9 | 19 |
| GMT±standard deviation | 2,613.48±1.7 6 | 155.30±3.0 5 | 3,089.00±1.6 1 | 187.38±3.0 2 | 10.71±1. 71 |
| 95% CI (lower limit, upper limit) | (2,005.78, 3,405.31) | (70.02, 344.47) | (2,435.08, 3,918.53) | (80.13, 438.13) | (8.28, 13.86) |
| P-value vs. placebo | <0.0001 t | <0.0001 t | <0.0001 t | <0.0001 t | |
| P-value vs. RBD nanoparticle 10 ug AS03 formulation | | <0.0001 t | 0.3346 t | | |
| P-value vs. RBD nanoparticle 25 ug AS03 formulation | | | | <0.0001 t | |
| | | | | | |
| GMFR±standard deviation | 221.31±2.18 | 14.19±2.58 | 250.39±1.85 | 14.30±2.96 | 1.00±1.7 6 |
| 95% CI (lower limit, upper limit) | (153.81, 318.44) | (7.21, 27.92) | (184.42, 339.95) | (6.20, 32.98) | (0.76, 1.31) |
| P-value vs. placebo | <0.0001 t | <0.0001 t | <0.0001 t | <0.0001 t | |
| P-value vs. RBD nanoparticle 10 ug AS03 formulation | | <0.0001 t | 0.5935 t | | |
| P-value vs. RBD nanoparticle 25 ug AS03 formulation | | | | <0.0001 t | |
| | | | | | |

| Visit 7 (4 weeks after second vaccination) | | | | | |
|---|---|---|---|---|---|
| n | 20 | 10 | 18 | 9 | 19 |
| GMT±standard deviation | 2,039.93±1.7 0 | 132.64±2.7 6 | 2,366.09±1.6 0 | 167.21±2.6 9 | 14.37±1. 71 |
| 95% CI (lower limit, upper limit) | (1,589.58,2,6 17.87) | (64.23,273. 91) | (1,871.90,2,9 90.75) | (78.05,358. 21) | (11.10,18 .60) |
| P-value vs. placebo | <0.0001 t | <0.0001 t | <0.0001 t | <0.0001 t | |
| P-value vs. RBD nanoparticle 10 ug AS03 formulation | | <0.0001 t | 0.3717 t | | |
| P-value vs. RBD nanoparticle 25 ug AS03 formulation | | | | <0.0001 t | |
| | | | | | |
| GMFR±standard deviation | 172.74±2.03 | 12.12±2.37 | 191.79±1.88 | 12.76±2.74 | 1.34±1.7 7 |
| 95% CI (lower limit, upper limit) | (124.00, 240.64) | (6.54, 22.47) | (140.21, 262.34) | (5.88, 27.70) | (1.02, 1.76) |
| P-value vs. placebo | <0.0001 t | <0.0001 t | <0.0001 t | <0.0001 t | |
| P-value vs. RBD nanoparticle 10 ug AS03 formulation | | <0.0001 t | 0.6350 t | | |
| P-value vs. RBD nanoparticle 25 ug AS03 formulation | | | | <0.0001 t | |

As shown in Table 8, after the first and second vaccinations with the RBD nanoparticle AS03 formulations of the present disclosure, the GMT of IgG antibodies significantly increased relative to the baseline, indicating significant immunogenicity. For both the low and high doses of the RBD nanoparticle AS03 formulations, the GMT significantly increased relative to the baseline two weeks after the second vaccination. Additionally, it was confirmed that the increased GMT was maintained even four weeks after the second vaccination, and that a higher GMT was observed in the high dose of the RBD nanoparticle AS03 formulation relative to the low dose. In addition, at the same time period, for example, as seen in the results for two weeks after the second vaccination, the RBD nanoparticle AS03 formulations showed a significantly higher GMT of IgG antibodies by up to 16 times or more relative to the single administration of RBD nanoparticles. The above numerical value is a significantly high level of antibody titer compared to the serum of convalescent patients from the National Institute for Biological Standards and Control (NIBSC) in the UK (WHO Reference Panel, First WHO International Reference Panel for anti-SARS-CoV-2 immunoglobulin, NIBSC code: 20/268), confirming that the vaccine composition of the present disclosure is significantly effective as a vaccine targeting SARS-CoV-2.

Based on the above results, it can be seen that the RBD nanoparticle AS03 formulations had higher immunogenicity than the single administration of RBD nanoparticles, and thus the RBD nanoparticle AS03 formulations can be effectively used as an active ingredient of vaccine compositions for preventing or treating SARS-CoV-2 infection.

### 1-2. Confirmation of Immunogenicity by Geometric Mean Titer and Seroconversion Rate of Neutralizing Antibodies

The RBD nanoparticle AS03 formulations (10 µg or 25 µg) and RBD nanoparticles alone (10 µg or 25 µg) were administered to the experimental subjects described in Table 7 above, and the geometric mean titer and seroconversion rate of IgG antibodies against SARS-CoV-2-RBD were confirmed by ELISA. Table 9 shows the geometric mean titer and seroconversion rate of neutralizing antibodies for the administration of RBD nanoparticle AS03 formulations (10 µg or 25 µg) and RBD nanoparticles alone (10 µg or 25 µg). Neutralizing antibodies are antibodies that bind to viruses and neutralize them so that they cannot penetrate cells, thus neutralizing antibodies are an important indicator for confirming immunogenicity.

**[Table 9]**

| | RBD nanoparticle 10 ug AS03 formulation (N=20) | RBD nanoparticle 10 ug (N=10) | RBD nanoparticle 25 ug AS03 formulation (N=18) | RBD nanoparticle 25 ug (N=9) | Placebo (N=19) |
|---|---|---|---|---|---|
| | | | | | |

| Geometric Mean Titer (GMT) and Seroconversion Rate Baseline | | | | | |
|---|---|---|---|---|---|
| n | 20 | 10 | 18 | 9 | 19 |
| GMT±standa rd deviation | 19.13±1.49 | 21.01±1.58 | 20.97±1.53 | 18.85±1.36 | 19.95±1.63 |
| 95% CI (lower limit, upper limit) | (15.89, 23.02) | (15.16, 29.12) | (16.96, 25.91) | (14.86, 23.91) | (15.78, 25.21) |
| P-value vs. placebo | 0.7685 t | 0.7832 t | 0.7434 t | 0.7516 t | |
| P-value vs. RBD nanoparticle 10 ug AS03 formulation | | 0.5659 t | 0.4962 t | | |
| P-value vs. RBD nanoparticle 25 ug AS03 formulation | | | | 0.5121 t | |

| Visit 4 (4 weeks after first vaccination) | | | | | |
|---|---|---|---|---|---|
| n | 20 | 10 | 18 | 9 | 19 |
| GMT±standa rd deviation | 91.95±3.03 | 32.79±2.45 | 90.08±2.78 | 25.76±1.91 | 24.22±2.03 |
| 95% CI (lower limit, upper limit) | (54.70, 154.58) | (17.28, 62.23) | (54.15, 149.85) | (15.68, 42.35) | (17.23, 34.05) |
| P-value vs. placebo | <0.0001 t | 0.3256 t | <0.0001 t | 0.8264 t | |
| P-value vs. RBD nanoparticle 10 ug AS03 formulation | | 0.0167 t | 0.9532 t | | |
| P-value vs. RBD nanoparticle 25 ug AS03 formulation | | | | 0.0027 t | |
| | | | | | |
| GMFR±stand | 4.81±3.23 | 1.56±2.42 | 4.30±3.03 | 1.37±1.68 | 1.21±2.52 |
| ard deviation | | | | | |
| 95% CI (lower limit, upper limit) | (2.77, 8.33) | (0.83, 2.94) | (2.48, 7.45) | (0.92, 2.04) | (0.78, 1.90) |
| P-value vs. placebo | 0.0003 t | 0.4862 t | 0.0006 t | 0.7241 t | |
| P-value vs. RBD nanoparticle 10 ug AS03 formulation | | 0.0126 t | 0.7642 t | | |
| P-value vs. RBD nanoparticle 25 ug AS03 formulation | | | | 0.0012 t | |
| | | | | | |

| Visit 6 (2 weeks after second vaccination) | | | | | |
|---|---|---|---|---|---|
| n | 20 | 10 | 18 | 9 | 19 |
| GMT±standa rd deviation | 1,787.58±2.9 0 | 83.47±4.47 | 1,984.01±2.3 1 | 70.09±4.58 | 19.47±1.54 |
| 95% CI (lower limit, upper limit) | (1,086.58, 2,940.83) | (28.62, 243.48) | (1,309.60, 3,005.71) | (21.76, 225.80) | (15.82, 23.96) |
| P-value vs. placebo | <0.0001 t | 0.0134 t | <0.0001 t | 0.0362 t | |
| P-value vs. RBD nanoparticle 10 ug AS03 formulation | | <0.0001 t | 0.7408 t | | |
| P-value vs. RBD nanoparticle 25 ug AS03 formulation | | | | <0.0001 t | |
| | | | | | |
| GMFR±stand ard deviation | 93.45±3.06 | 3.97±4.09 | 94.63±2.46 | 3.72±4.36 | 0.98±1.44 |
| 95% CI (lower limit, upper limit) | (55.34, 157.79) | (1.45, 10.88) | (60.54, 147.93) | (1.20, 11.53) | (0.82, 1.16) |
| P-value vs. placebo | <0.0001 t | 0.0117 t | <0.0001 t | 0.0263 t | |
| P-value vs. RBD nanoparticle 10 ug AS03 formulation | | <0.0001 t | 0.9699 t | | |
| P-value vs. RBD nanoparticle 25 ug AS03 formulation | | | | <0.0001 t | |
| | | | | | |

| Visit 7 (4 weeks after second vaccination) | | | | | |
|---|---|---|---|---|---|
| n | 20 | 10 | 18 | 9 | 19 |
| GMT±standa rd deviation | 1,315.13±2.8 7 | 68.52±3.85 | 1,574.05±2.8 7 | 69.18±4.89 | 19.24±1.46 |
| 95% CI (lower limit, upper limit) | (802.48, 2,155.28) | (26.12, 179.79) | (932.54, 2,656.85) | (20.43, 234.28) | (16.03, 23.09) |
| P-value vs. placebo | <0.0001 t | 0.0157 t | <0.0001 t | 0.0425 t | |
| P-value vs. RBD nanoparticle 10 ug AS03 formulation | | <0.0001 t | 0.6030 t | | |
| P-value vs. RBD nanoparticle 25 ug AS03 formulation | | | | <0.0001 t | |
| | | | | | |
| GMFR±stand ard deviation | 68.75±2.91 | 3.26±4.15 | 75.08±3.50 | 3.67±4.97 | 0.96±1.60 |
| 95% CI (lower limit, upper limit) | (41.72, 113.30) | (1.18, 9.02) | (40.29, 139.90) | (1.07, 12.58) | (0.77, 1.21) |
| P-value vs. placebo | <0.0001 t | 0.0249 t | <0.0001 t | 0.0376 t | |
| P-value vs. RBD nanoparticle 10 ug AS03 formulation | | <0.0001 t | 0.8163 t | | |
| P-value vs. | | | | <0.0001 t | |
| RBD nanoparticle 25 ug AS03 formulation | | | | | |

As shown in Table 9, after the first and second vaccinations with the RBD nanoparticle AS03 formulations of the present disclosure, the GMT of neutralizing antibodies significantly increased relative to the baseline, indicating significant immunogenicity. For both the low and high doses of the RBD nanoparticle AS03 formulations, the GMT significantly increased relative to the baseline two weeks after the second vaccination. Additionally, it was confirmed that the increased GMT was maintained even four weeks after the second vaccination, and that a higher GMT was observed in the high dose of RBD nanoparticle AS03 formulation relative to the low dose. In addition, at the same time period, for example, as seen in the results for two weeks after the second vaccination, the RBD nanoparticle AS03 formulations showed a significantly higher GMT of neutralizing antibodies by up to 28 times or more relative to the single administration of RBD nanoparticles. The above numerical value is a significantly high level of antibody titer compared to the serum of convalescent patients from the National Institute for Biological Standards and Control (NIBSC) in the UK (WHO Reference Panel, First WHO International Reference Panel for anti-SARS-CoV-2 immunoglobulin, NIBSC code: 20/268), confirming that the vaccine composition of the present disclosure is significantly effective as a vaccine targeting SARS-CoV-2.

Based on the above results, it can be seen that the RBD nanoparticle AS03 formulations had higher immunogenicity than the single administration of RBD nanoparticles, and thus the RBD nanoparticle AS03 formulations can be effectively used as an active ingredient of vaccine compositions for preventing or treating SARS-CoV-2 infection.

### Example 2. Use of RBD Nanoparticle AS03 Formulation as Vaccine Booster Composition and Confirmation of Formation of Immunity formation

The RBD nanoparticle 25 µg-AS03 formulation prepared in the Preparation Example was used as a vaccine booster composition to determine whether immunity was formed in subjects.

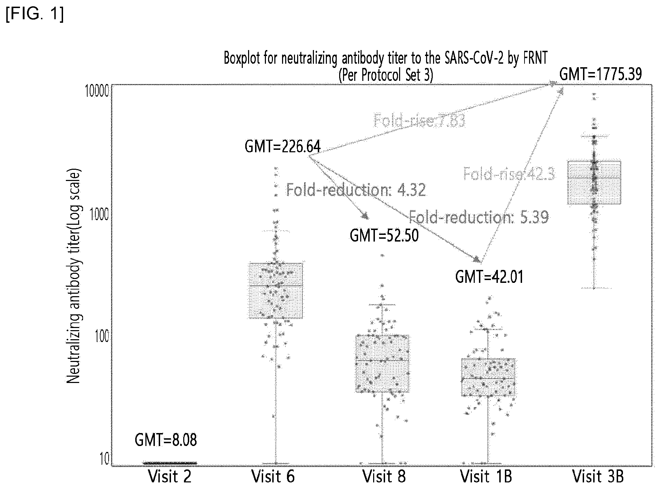

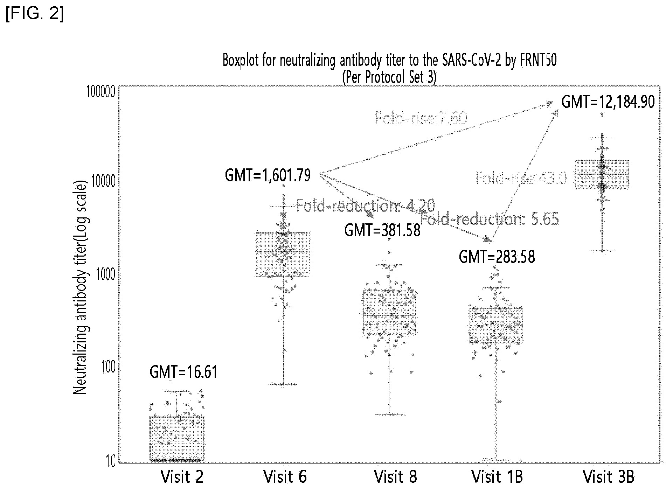

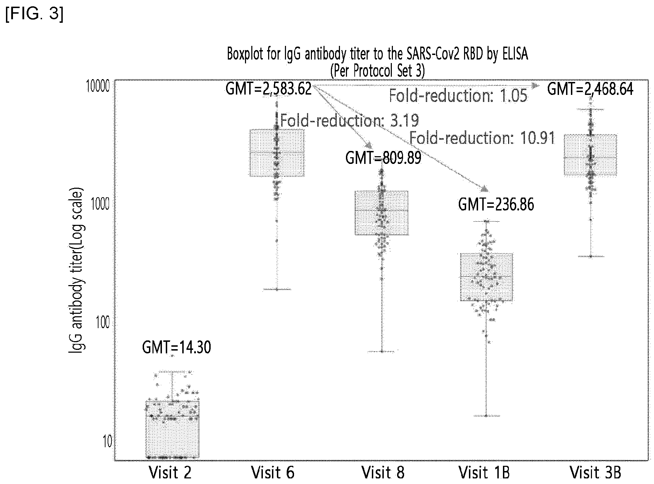

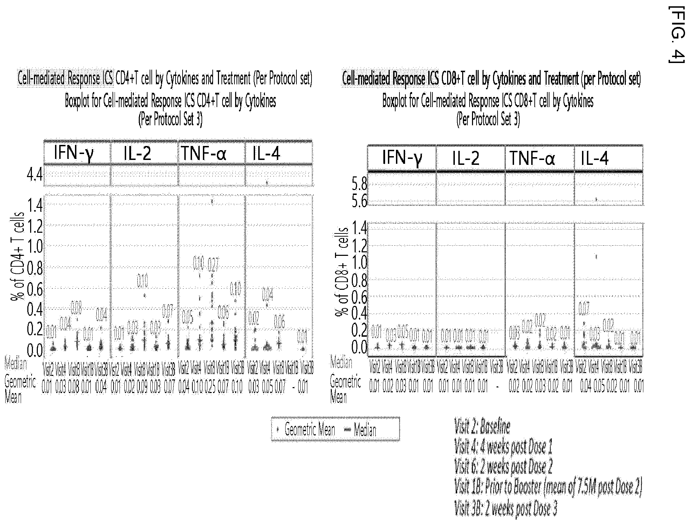

The study was conducted on 81 adults aged 19 to 85 years. The first and second vaccinations were performed in the same manner as in Example 1, and the third vaccination was performed approximately 7.5 months after the second vaccination. The results of confirming the immune response to the Wuhan strain at the baseline (Visit 2), 2 weeks after the second vaccination (Visit 6), 3 months after the second vaccination (Visit 8), 7.5 months after the second vaccination (before booster shot vaccination; Visit 1B), and 2 weeks after the booster shot vaccination (Visit 3B) are shown in FIGS. 1 to 4, respectively. The FRNT result of the group of 2 weeks after the booster shot vaccination (Visit 3B) showed an increased neutralizing antibody titer by 7.83, 42.3-fold compared to 3 months after the second vaccination (Visit 8) and 7.5 months after the second vaccination (before booster shot vaccination; Visit 1B). In the case of cellular immunogenicity (CD4+ T cell), cytokine (IFN-r, IL-2, TNF-a, IL-4)-secreting T cells were observed at a relatively higher level compared to the group of 7.5 months after vaccination (Visit 1B). Further, there was no significant difference in the case of cellular immunogenicity (CD8+ T cell).

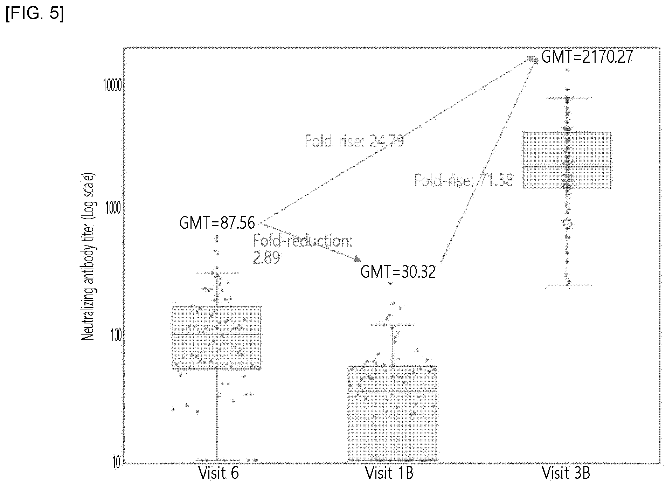

Additionally, the results of the immune response to the Omicron variant strain at 2 weeks after the second vaccination (Visit 6), 7.5 months after the second vaccination (before booster shot vaccination; Visit 1B), and 2 weeks after the booster shot vaccination (Visit 3B) are shown in FIG. 5. As a result of calculating the neutralizing antibody titer at 2 weeks after the booster shot vaccination (Visit 3B), it was confirmed that the neutralizing antibody titer increased by 24.79 and 71.58-fold, respectively, compared to 2 weeks after the second vaccination (Visit 6) and 7.5 months after the second vaccination (before booster shot vaccination; Visit 1B).

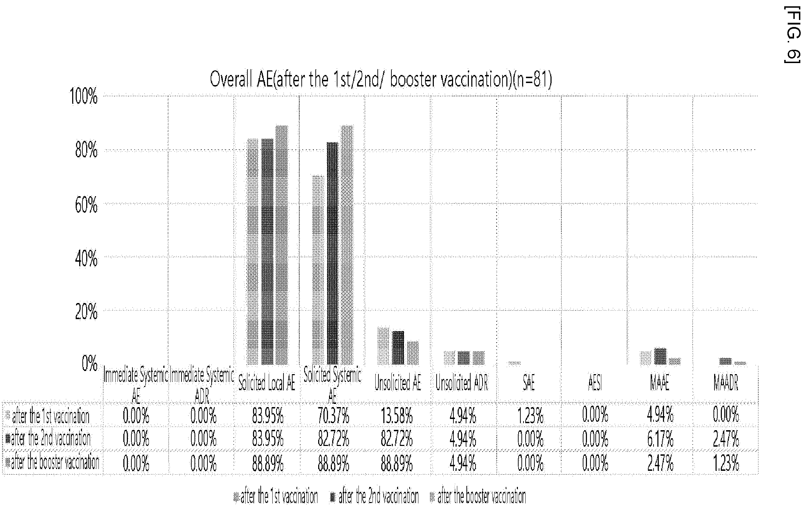

As a result of observing adverse events that occurred after the booster shot vaccination, it was confirmed that the occurrence of unspecified adverse events rather decreased (FIG. 6).

### Example 3. Use of RBD Nanoparticle AS03 Formulation as Vaccine Booster Composition and Additional Verification of Formation of Immunity Against Variant Strains (Mouse Experiment)

### Example 3-1. Experimental Materials and Methods

The RBD nanoparticle 25 µg-AS03 formulation prepared in the Preparation Example was used as a vaccine booster composition to determine whether immunity was formed against the variant SARS-CoV-2.

5-week-old hACE2 transgenic mice were purchased from Jackson Laboratory and used in the experiment. The mice were divided into immunization groups as shown in Table 10 below and tested. Immunity was formed at 3-week intervals. In the table below, GBP510-Wu (RBD-NP-WT) was identical to the RBD nanoparticle prepared in Preparation Example 1, GBP510-Beta (RBD-NP-Beta) was prepared by replacing the SARS-CoV-2 RBD sequence of RBD-Component A of Preparation Example 1 with the RBD sequence of the Beta virus, and GBP510-Bivalent was administered in a combination of GBP510-Wu and GBP510-Beta.

The Wuhan strain and Alpha, Beta, Gamma, and Delta variant strains were used in the challenge test. The dosage for each group, *etc.* is listed in Table 12.

**[Table 10]**

| | First Immunizatio n | Second Immunizatio n | Third Immunization | Challenge Test Viruses |
|---|---|---|---|---|
| Group 1 | GBP510-Wu | GBP510-Wu | | Wuhan, Alpha, Beta, Gamma, Delta |
| Group 2 | GBP510-Wu | GBP510-Wu | GBP510-Wu | |
| Group 3 | GBP510-Wu | GBP510-Wu | GBP510-Beta | |
| Group 4 | GBP510-Wu | GBP510-Wu | GBP510-Bivalent | |

**[Table 11]**

| SARS-CoV-2 variant (WHO Nomenclature ) | Information on Viruses |
|---|---|
| Wuhan (WT) | BetaCoV/Korea/KCDC03/2020 |
| Alpha | hCoV-19/Korea/KDCA51463/2021; GRY: B.1.1.7 |
| Beta | hCoV-19/Korea/KDCA55905/2021; GH: B.1.351 |
| Gamma | hCoV-19/Korea/KDCA95637/2021; GR: P.1 |
| Delta | hCoV-19/Korea/KDCA5439/2021; G: B.1.617.2 |

**[Table 12]**

| **Groups** | **Sample** | **Antigen conc. (mcg/dose)** | **No. of animals** | **Adjuvant** | **Challenge strain** |
|---|---|---|---|---|---|
| 1 | Vehicle | 0 | 10 | - | Wild-type SARS-CoV-2 |
| 2 | RBD-NP-WT x2 | 5 | 10 | AS03 | |
| 3 | RBD-NP-WT x 3 | 5 | 10 | AS03 | |
| 4 | RBD-NP-WT x 2 + RBD-NP-Beta x 1 | 5 | 10 | AS03 | |
| 5 | RBD-NP-WT x 2 + Bivalent x 1 | 5(Bivalent; 5 of RBD-NP-WT and 5 of RBD-NP-Beta) | 10 | AS03 | |

| **Groups** | **Sample** | **Antigen conc. (mcg/dose)** | **No. of animals** | **Adjuvant** | **Challenge strain** |
|---|---|---|---|---|---|
| 1 | Vehicle | 0 | 10 | - | Alpha variant |
| 2 | RBD-NP-WT x 2 | 5 | 10 | AS03 | |
| 3 | RBD-NP-WT x 3 | 5 | 10 | AS03 | |
| 4 | RBD-NP-WT x 2 + RBD-NP-Beta x 1 | 5 | 10 | AS03 | |
| 5 | RBD-NP-WT x 2 + Bivalent x 1 | 5(Bivalent; 5 of RBD-NP-WT and 5 of RBD-NP-Beta) | 10 | AS03 | |
| 1 | Vehicle | 0 | 10 | - | Beta variant |
| 2 | RBD-NP-WT x 2 | 5 | 10 | AS03 | |
| 3 | RBD-NP-WT x 3 | 5 | 10 | AS03 | |
| 4 | RBD-NP-WT x 2 + RBD-NP-Beta x 1 | 5 | 10 | AS03 | |
| 5 | RBD-NP-WT x 2 + Bivalent x 1 | 5(Bivalent; 5 of RBD-NP-WT and 5 of RBD-NP-Beta) | 10 | AS03 | |

| **Groups** | **Sample** | **Antigen conc. (mcg/dose)** | **No. of animals** | **Adjuvant** | **Challenge strain** |
|---|---|---|---|---|---|
| 1 | Vehicle | 0 | 10 | - | Gamma variant |
| 2 | RBD-NP-WT x 2 | 5 | 10 | AS03 | |
| 3 | RBD-NP-WT x 3 | 5 | 10 | AS03 | |
| 4 | RBD-NP-WT x 2 + RBD-NP-Beta x 1 | 5 | 10 | AS03 | |
| 5 | RBD-NP-WT x 2 + Bivalent x 1 | 5(Bivalent; 5 of RBD-NP-WT and 5 of RBD-NP-Beta) | 10 | AS03 | |

| **Groups** | **Sample** | **Antigen conc. (mcg/dose)** | **No. of animals** | **Adjuvant** | **Challenge strain** |
|---|---|---|---|---|---|
| 1 | Vehicle | 0 | 10 | - | Delta variant |
| 2 | RBD-NP-WT x 2 | 5 | 10 | AS03 | |
| 3 | RBD-NP-WT x 3 | 5 | 10 | AS03 | |
| 4 | RBD-NP-WT x 2 + RBD-NP-Beta x 1 | 5 | 10 | AS03 | |
| 5 | RBD-NP-WT x 2 + Bivalent x 1 | 5(Bivalent; 5 of RBD-NP-WT and 5 of RBD-NP-Beta) | 10 | AS03 | |

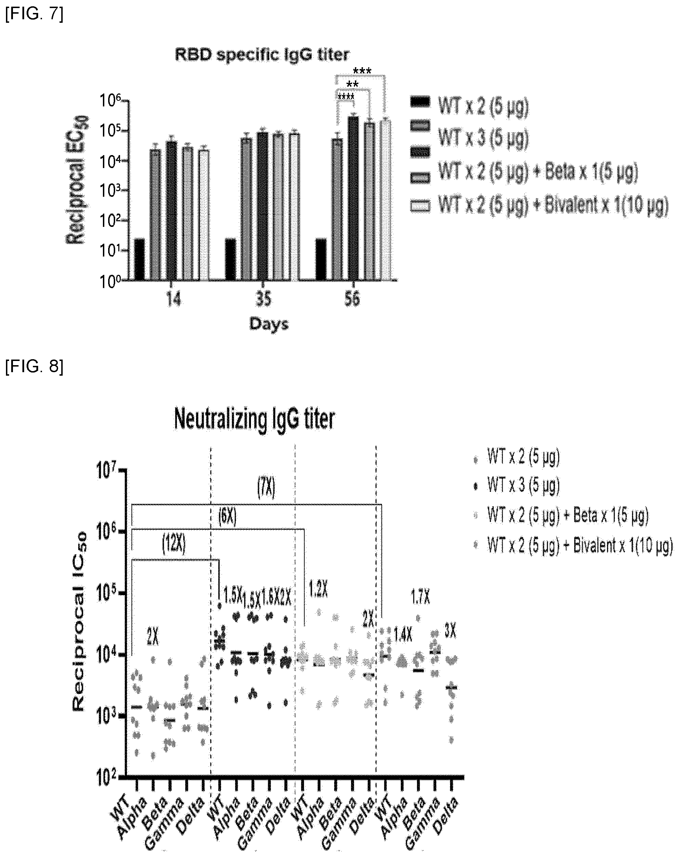

Blood collection was performed every 2, 5, and 8 weeks, and the serum samples collected at each time point were analyzed to confirm the immunogenicity of the GBP510 vaccine. The experimental results are shown in FIG. 7. Additionally, a CPE-based neutralization test was performed using serum samples collected at week 8 after the first immunization, and the results are shown in FIG. 8.

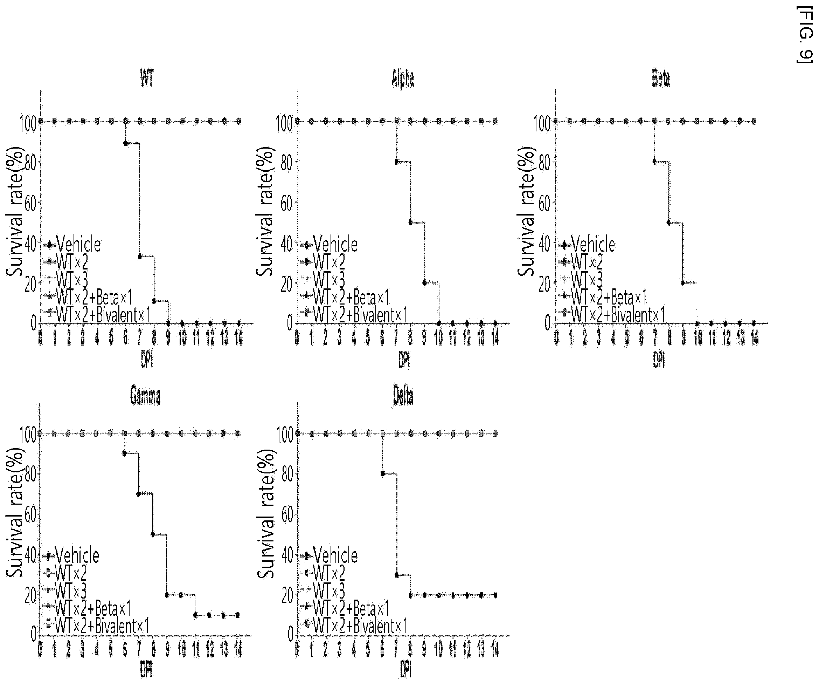

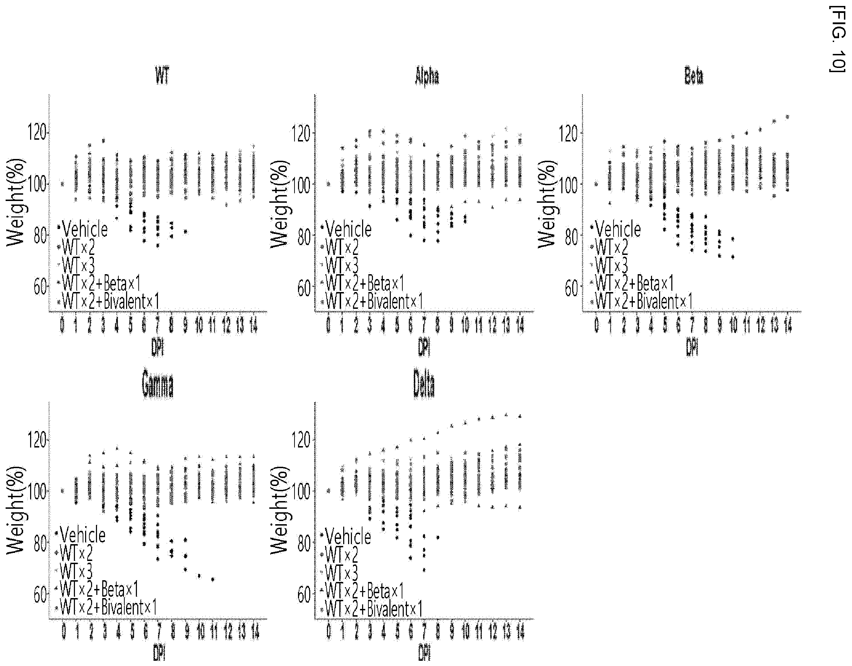

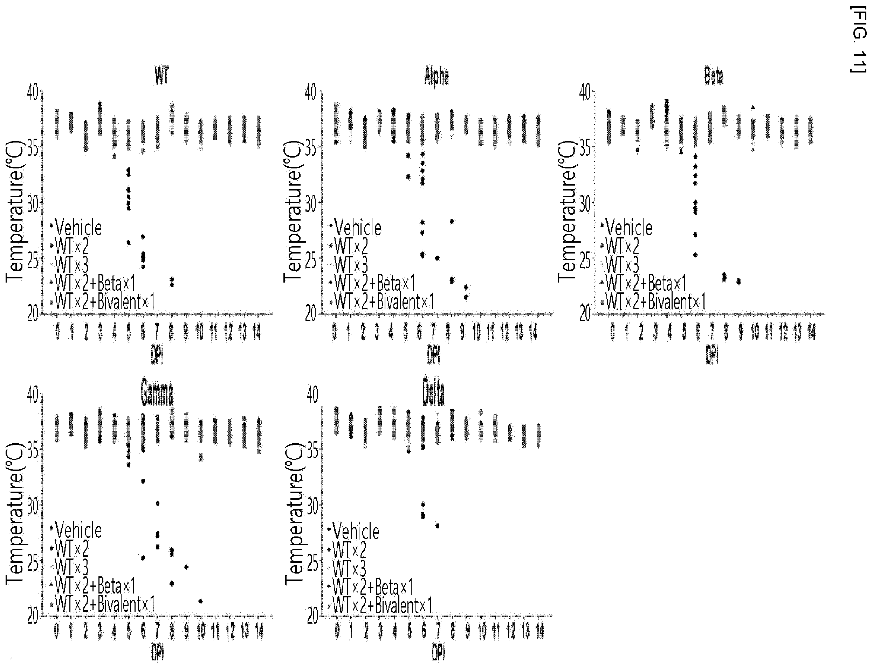

One week after the third immunization, the mice were nasally infected with the SARS-CoV-2 Wuhan and variant strains at a dose of 1×10⁴ PFU/head at week 9, and the changes in survival rate, body weight, and body temperature were observed for 14 days, and the results are shown in FIGS. 9 to 11, respectively.

Additionally, lung tissues were harvested from the hACE2-Tg mice, total RNA was extracted, and qRT-PCR was performed. The PCR was performed under PCR conditions of denaturation at 95°C for 30 seconds, followed by 45 cycles of denaturation at 95°C for 5 seconds and annealing at 60°C for 20 seconds. The primer information used is as follows.
E gene: forward primer, 5'-ACAGGTACGTTAATAGTTAATAGCGT-3';
E gene: reverse primer, 5'-ATATTGCAGCAGTACGCACACA-3';
probe [FAM]5'-ACACTAGCCATCCTTACTGCGCTTCG-3'[BHQ1];
RdRp gene: forward primer 5'-ATGAGCTTAGTCCTGTTG-3';
RdRp gene: reverse primer 5'-CTCCCTTTGTTGTGTTGT-3';
probe [HEX]5'-AGATGTCTTGTGCTGCCGGTA-3'[BHQ1]

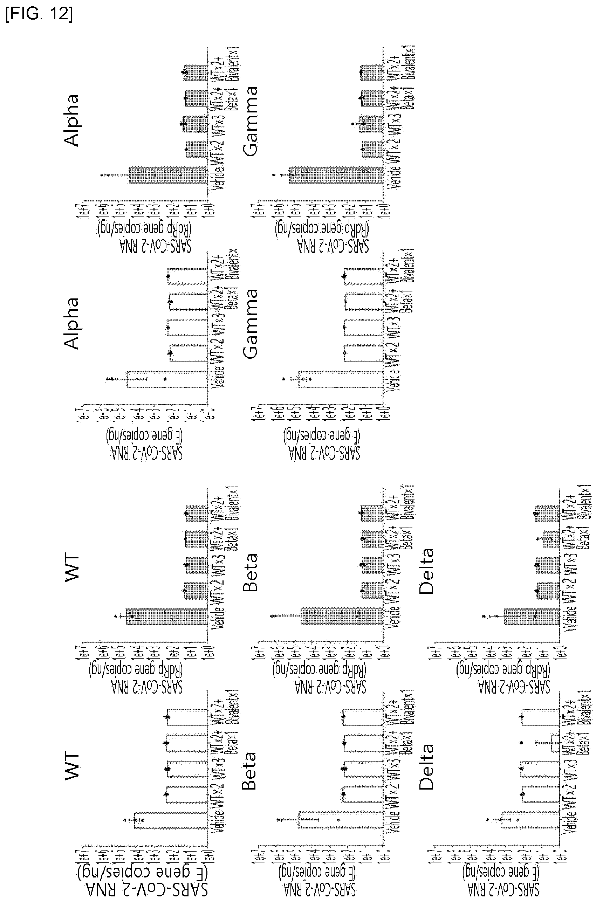

After completion of the reaction cycles, the temperature was increased from 65 to 95°C at a rate of 0.2°C/15 seconds. Fluorescence was measured every 5 seconds to generate a melting curve. A sample without template was used as a control. Bio-Rad CFX Manager, version 2.1 software was used, and the viral burden was obtained by calculating the absolute copy number of viral RNA by comparison with a standard cDNA template of viral RNA, and then expressed as the number of copies of viral RNA per total RNA nanogram. The results are shown in FIG. 12.

### Example 3-2. Experimental Results

As shown in FIG. 7, it was confirmed that the antigen-specific total antibody titer was higher than the control group at 2, 5, and 8 weeks, and that a higher antibody titer was induced relative to the high-level second immunization group after the third immunization, and the difference therebetween was statistically significant.

As shown in FIG. 8, it was confirmed that the neutralizing antibody titer increased by 12-fold, 6-fold, and 7-fold, respectively, after the third immunization with GBP510-Wu, GBP510-Beta, and GBP510-Bivalent compared to the second immunization.

As shown in FIGS. 9 to 11, the results of the challenge test showed that not only did the third immunization group survive the virus infection, but also the second immunization group survived as well, and the protective immunity formed by GBP510 immunization was clearly confirmed since the changes in body weight and body temperature were not significant.

As shown in FIG. 12, it was confirmed that the viral load was significantly lowered not only in the third immunization group but also in the second immunization group.

### Example 4. Viral load, Histopathology and Cytokine Analysis in Bronchoalveolar Lavage Fluid (BALF) and Lung Tissues

### Example 4-1. Experimental Materials and Methods

The challenge test was performed by administering the SARS-CoV-2 Wuhan strain or Delta variant to the hACE2 TG mice at a dose of 1 × 10⁴ PFU/mouse on 63 days after the first vaccination according to the same schedule as Example 3. 5 days after the challenge test, lung tissues were harvested, and viral load, histopathology, and cytokine analysis were performed.

The information on mice used in Example 4 is as follows.

**[Table 13]**

| **Groups** | **Sample** | **Antigen conc. (mcg/dose)** | **No. of animals** | **Adjuvant** | **Challenge strain** |
|---|---|---|---|---|---|
| 1 | Vehicle | 0 | 3 | - | Wild-type |
| 2 | RBD-NP-WT x 2 | 5 | 3 | AS03 | SARS-CoV-2 |
| 3 | RBD-NP-WT x 3 | 5 | 3 | AS03 | |
| 4 | RBD-NP-WT x 2 + RBD-NP-Beta x 1 | 5 | 3 | AS03 | |

| **Groups** | **Sample** | **Antigen conc. (mcg/dose)** | **No. of animals** | **Adjuvant** | **Challenge strain** |
|---|---|---|---|---|---|
| 1 | Vehicle | 0 | 3 | - | Delta variant |
| 2 | RBD-NP-WT x 2 | 5 | 3 | AS03 | |
| 3 | RBD-NP-WT x 3 | 5 | 3 | AS03 | |
| 4 | RBD-NP-WT x 2 + RBD-NP-Beta x 1 | 5 | 3 | AS03 | |

Histopathological analysis was performed using large lung lobes perfused with 10% neutral buffered formalin. 5 days after SARS-CoV-2 virus infection, lung tissues were embedded in paraffin, 4-µm sections were prepared, stained with hematoxylin and eosin, and observed under a microscope. Pulmonary inflammation and goblet cell hyperplasia were graded using a previously known semiquantitative scoring system. Peribronchial and perivascular inflammation were assessed as a blind experiment based on a 5-point grading system to grade the level of inflammatory cell infiltration: 0, normal; 1, low-grade cellular influx, no histopathology; 2, low- to moderate-grade cellular influx, low-grade tissue damage; 3, moderate-grade cellular influx, low-grade tissue damage; 4, moderate-to high-grade cellular influx, marked tissue damage; 5, high-grade cellular influx, significant histopathology.

Inflammation-related cytokine analysis was performed using bronchoalveolar lavage fluid (BALF) samples collected on day 5 after SARS-CoV-2 virus infection. Specifically, BALF was sampled with 300 µL of PBS, and the samples were diluted 1:1 with an assay buffer of the mouse inflammation assay panel. Premix beads were added to all of the wells from well plates and incubated at room temperature for 1 hour and 30 minutes, then the supernatant was discarded, and 200 µL of wash buffer was added to all of the wells from the well plates and washed. The supernatant was discarded and the washing step was repeated twice. Subsequently, the samples were incubated at room temperature for 1 minute, and then 25 µL of a detection antibody was added to all of the wells and incubated at room temperature for 40 minutes. Thereafter, 25 µL of SA-PE reagent was added to all of the wells and incubated at room temperature for 20 to 30 minutes. 200 µL of a wash buffer was added to all of the wells from the well plates and incubated at room temperature for 1 minute. The supernatant was discarded and the washing step was repeated twice. 150 µL of PBS was added to all of the wells and the entire sample was transferred to a tube. After adding 150 µL of PBS to all of the wells, 400 µL of raw beads were prepared and mixed with fluorescent beads. Data analysis was performed using LEGENDplex^{™} Data Analysis Software according to the manufacturer's instructions.

After the virus challenge test, lung tissues were extracted and subjected to the plaque reduction neutralization test (PRNT) analysis. Specifically, after the virus challenge test, lung tissues were extracted, homogenized, and centrifuged at 5,000 rpm, 4°C for 5 minutes to collect the supernatant. Vero E6 cells were seeded at 2X10⁵ cells per well in 12-well plates and cultured in DMEM containing 10% fetal bovine serum. After cell confluency, the cells were washed with serum-free DMEM and infected with 150 ul of serial-diluted supernatant containing SARS-CoV2 of the lung tissues. The cells were further incubated at 37°C for 30 minutes in a CO₂ incubator with intermittent shaking every 15 minutes. After aspiration of the inoculum, the cells in the 12-well plate were overlaid with 1% low-melting agarose in 1X MEM containing 2% FBS. Plates were incubated for 48 to 72 hours until plaques were observed, and then, the cells were fixed with 4% neutral buffered formalin for 1 hour at room temperature and stained with 0.5% crystal violet. Virus titers were calculated as follows: PFU/ml = (average number of plaques/volume [ml] of virus added) x dilution factor. The final titer of infectious SARS-CoV-2 in lung tissue was expressed as infectious SARS-CoV-2 plaques per gram of lung tissue.

### Example 4-2. Experimental Results

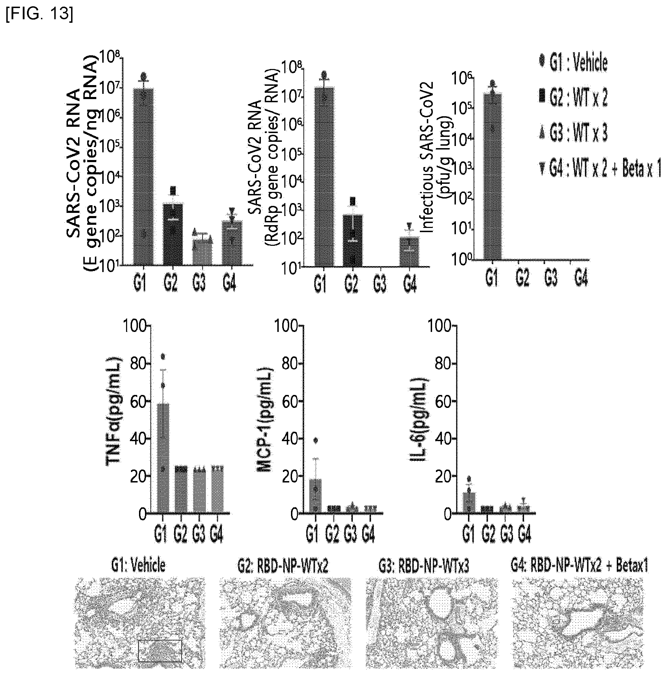

The experimental results are shown in FIG. 13. As a result of genomic DNA analysis and plaque analysis, it was observed that the control group (vehicle) had a high level of viral load, while the vaccinated groups had a significantly low or undetectable level of viral load. Vaccination showed protective efficacy against not only the wild type (Wuhan strain) but also the Delta variant strain.

Additionally, the results of analysis on the inflammation level of cytokines secreted in BALF samples showed that inflammatory cytokines were reduced by vaccination. This suggests that vaccination suppresses the progression of inflammation. Additionally, TNF-a and IL-6, which are important indicators of inflammation, were rarely detectable, and MCP-1 chemokine, which is associated with the recruitment of inflammatory monocytes, was detected in a significantly low level in the vaccinated groups. This suggests that vaccination can protect individuals from the secretion of inflammatory cytokines.

As a result of histopathological analysis, inflammation in lung tissue was detected at a lower level in the vaccinated groups administered 2 to 3 times than in the control group, and no toxicity of the vaccine composition was detected.

### Example 5. Use of RBD Nanoparticle AS03 Formulation as Vaccine Booster Composition and Additional Verification of Formation of Immunity Against Variant Strains (Primate Experiment)

The RBD nanoparticle 25 µg-AS03 formulation prepared in the Preparation Example was immunized using non-human primates (NHPs), and its effectiveness as a vaccine booster composition was verified.

### Example 5-1. Experimental Materials and Methods

Six rhesus macaques aged 2 to 5 years were assigned to each group. The RBD nanoparticle vaccine was administered intramuscularly twice or three times at 28-day intervals. Blood samples were collected on days 14, 28, 42, 56, 70, and 77 after the first vaccination.

On day 77, the rhesus macaques were challenged intranasally and intratracheally with the Delta variant strain at a dose of 5 × 10⁵ TCID/head. Peripheral blood mononuclear cells (PBMCs) were isolated and the cell-mediated immune response was investigated by ELISpot analysis. The protective efficacy of the vaccine was evaluated by clinical signs and viral load.

Serum samples from a previous study (DOI: https://doi.org/10.1038/s41586-021-03530-2) immunized and challenged with RBD of SARS-CoV-2 and spike proteins were collected and used for neutralizing antibody titer test against the wild type (Wuhan strain) and Omicron pseudovirus. Five Rhesus macaques aged 3-9 years were assigned to each group. Immunization was performed on days 0 and 21, and serum samples were collected on day 42. Antigens used were AS03-adjuvanted RBD nanoparticles prepared in the aforementioned Preparation Example and HexaPro trimer (DOI: https://doi.org/10.1038/s41586-021-03530-2).

**[Table 14]**

| **Groups** | **Sample** | **Antigen conc. (mcg/dose)** | **No. of animals** | **Adjuvant** | **Challenge strain** |
|---|---|---|---|---|---|
| 1 | Vehicle | 0 | 6 | - | Delta variant |
| 2 | RBD-NP-WT x 2 | 25 | 6 | AS03 | |
| 3 | RBD-NP-WT x 3 | 25 | 6 | AS03 | |
| 4 | RBD-NP-WT x 2 + RBD-NP-Beta x 1 | 25 | 6 | AS03 | |

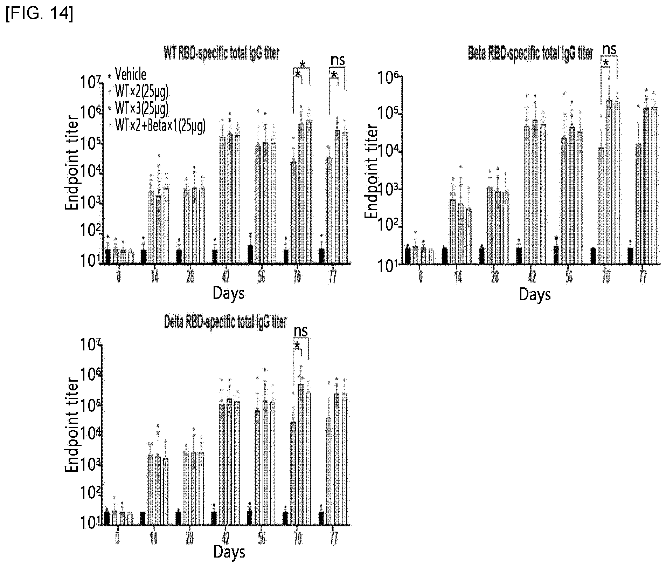

The measurement of total antibody titers by specific IgG ELISA was specifically performed as follows. The wild-type (Wuhan strain), Beta or Delta variant SARS-CoV-2 spike RBD recombinant proteins were prepared as 1 X DBPS (1 µg/mL), and 1 µg/mL of antigen was seeded in a 96-well plate at 100 µL/well and incubated overnight at 4°C, and then washed once with 300 µL/well of a wash solution, added to 350 µL/well of a blocking buffer and incubated for 2 to 3 hours at room temperature. After incubation, the blocking solution was discarded and the plate was dried. Heat-inactivated plasma was prepared from each group and stepwise diluted using a blocking buffer. The thus-diluted plasma was added to the wells and incubated for 1 hour at room temperature, and then washed three times with 300 µL/well of a wash solution. Thereafter, anti-macaque IgG HRP (1 µg/mL) was added and incubated at room temperature in a dark room for 1 hour, and then washed three times with 300 µL/well of a washing solution. Subsequently, a TMB substrate was added at 100 µL/well, and then the reaction was stopped by adding 100 µL/well of a stop solution. Thereafter, the absorbance was measured at 450 nm using a UV/VIS Microplate Reader (spectrophotometer). The antibody titer was calculated as follows: For each sample, the ELISA endpoint titer was calculated as the reciprocal of a plasma dilution that produces an absorbance value of 0.2 at 450 nm using a fourparameter logistic curve fit by Graphpad Prism software, and the Log₁₀ endpoint titer was obtained. The experimental results are shown in FIG. 14.

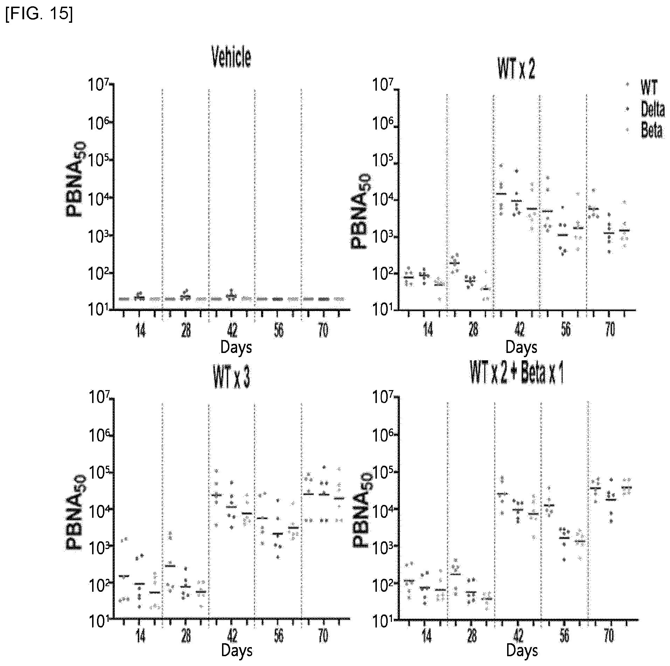

The measurement of neutralizing antibody titers (pseudovirus-based neutralization assays: wild type, Beta, Delta) was specifically performed as follows. SARS-CoV-2 expressing a luciferase reporter gene was prepared by co-transfecting HEK293T cells with packaging plasmid psPAX2, luciferase reporter plasmid pLenti-CMV Puro-Luc, and spike proteins expressing the wild type or variant pcDNA3.1-SARS CoV-2 SΔCT by Lipofectamine 2000. Pseudoviruses of SARS-CoV-2 were prepared using spike from the wild-type Wuhan strain (Wuhan/WIV04/2019, GISAID accession ID: EPI_ISL_402124), Beta variant strain (GISAID accession ID: EPI_ISL_712096), or Delta variant strain (GenBank Accession ID: QTW89558.1). 48 hours after infection, the supernatant containing pseudovirus was obtained by centrifugation and filtration through a 0.45 µm filter. In order to determine the neutralizing activity of the plasma samples, HEK293T-hACE2 cells were seeded at a density of 1.75 × 10⁴ cells/well in a 96-well plate and incubated overnight in a CO₂ incubator at 37°C. A 3-fold serial dilution of heat-inactivated plasma samples was prepared and mixed with 50 µL of pseudovirus. The mixture was incubated at 37°C for 1 hour and added to HEK293T-hACE2 cells. 48 hours after infection, the cells were lysed using the Steady-Glo Luciferase assay according to the manufacturer's instructions. The SARS-CoV-2 neutralizing titer was defined as the sample dilution point at which a 50% decrease of relative light units (RLU) was observed compared to the mean of the wells containing the virus control sample. The experimental results are shown in FIG. 15.

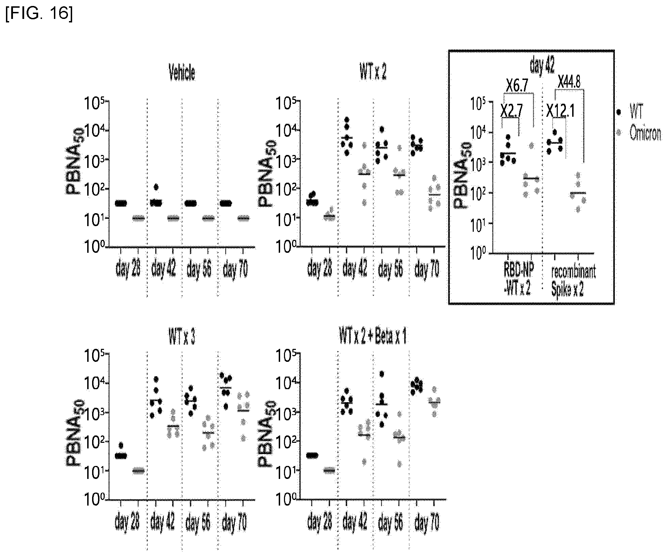

The measurement of neutralizing antibody titers for the wild-type (Wuhan strain) and Omicron (pseudovirus-based neutralization assay (PBNA)) was performed as follows. First, SARS-CoV-2 pseudoviruses expressing a luciferase reporter gene were prepared. HEK293T cells were transfected with plasmids encoding the spike glycoproteins from the wild-type and Omicron variant (B.1.1.529) using Lipofectamine 2000. One day after transfection, the cells were infected with VSV (G*ΔG-luciferase), and after 2 hours, the cells were washed five times with DMEM and added with a medium supplemented with anti-VSV-G antibodies. The pseudotyped viruses were harvested 18 to 24 hours after inoculation, purified by centrifugation at 2,500 × g for 5 min, filtered through a 0.45 µm cut-off membrane, concentrated 10 times through a 30 kDa cut-off membrane, seeded, and stored at -80°C. The HEK239T-hACE2 cells were seeded at a density of 4 × 10⁴ cells/well in a 96-well tissue culture plate and incubated overnight in a CO₂ incubator at 37°C. A 12-point 3-fold serial dilution of heat-inactivated plasma samples was prepared and mixed with the pseudovirus. The mixture was incubated at 37°C for 45 minutes and then added to the HEK239T-hACE2 cells. After 17 to 20 hours of infection, the cells were lysed with a One-Glo-EX substrate and incubated in a dark room for 5 to 10 minutes before reading. Relative luciferase units were plotted and normalized using Prism (GraphPad). IC₅₀ values were determined from the curve fit using a nonlinear regression of log(inhibitor) versus normalized reaction. The experimental results are shown in FIG. 16.

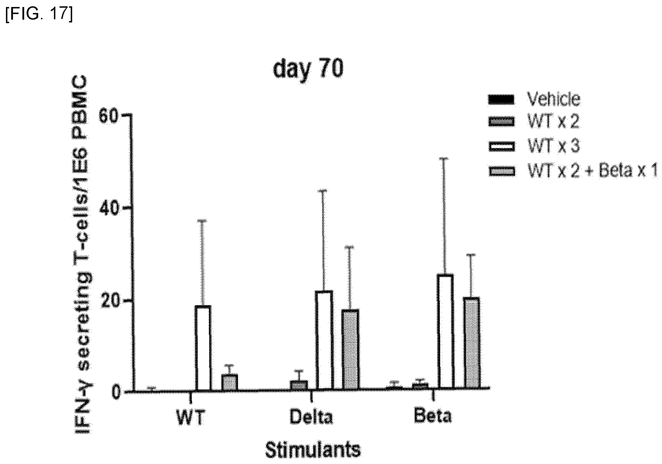

The assessment of cell-mediated immunity was specifically performed as follows. The ELISpot assay was performed using peripheral blood mononuclear cells (PBMCs). A peptide pool was constructed with 15 peptides overlapping 11 amino acids across the spike proteins of SARS-CoV-2 WT or variants. ELISpot plates were coated with 5 µg/well of a mouse anti-human IFN-γ monoclonal antibody and incubated overnight at 4°C. SARS-CoV-2 peptides were prepared and plated at a concentration of 1 µg/well, and 2 × 10⁵ cells/well of cells were added to the plates. The peptides and cells were incubated at 37°C for 18 to 24 hours. The positive control wells were cells containing phytohemagglutinin, and the negative control wells were cells containing media. The plates were washed with ELISpot wash buffer (11% 10x DPBS, 0.3% Tween 20 in 1 L of water) and incubated with biotinylated rabbit anti-human IFN-γ polyclonal antibodies (1 µg/mL) for 2 hours. The plates were washed twice and incubated with 2 µg/mL streptavidin-alkaline phosphatase for 2 hours. After the final wash, NBT/BCIP substrate solution was added for 7 minutes. The substrate was discarded, and the plates were washed with water and dried in the dark for 24 hours. The plates were scanned and counted using a Cellular Technologies Limited Immunospot Analyzer. The results of the experiment are shown in FIG. 17.

The measurement of viral load in lung tissues (subgenomic DNA analysis) was performed specifically as follows. Viral load was quantified from the bronchoalveolar lavage (BAL) fluid and nasal swab (NS). RNA extraction was performed on QIAcube HT using the IndiSpin QIAcube HT Pathogen Kit according to the manufacturer's instructions. Standard dilutions and extracted RNA samples were reverse transcribed using the SuperScript VILO Master Mix according to the manufacturer's instructions under the following cycling conditions: 25°C for 10 minutes, 42°C for 1 hour, and 85°C for 5 minutes. SARS-CoV-2 E gene subgenomic RNA (sgRNA) was processed by reverse transcription polymerase chain reaction. Primers and probes used are as follows.
probe [VIC]5'-ACACTAGCCATCCTTACTGCGCTTCG-3'[MGB];
E gene forward primer 5'-CGATCTCTTGTAGATCTGTTCTC-3';
E gene reverse primer 5'-ATATTGCAGCAGTACGCACACA-3'.

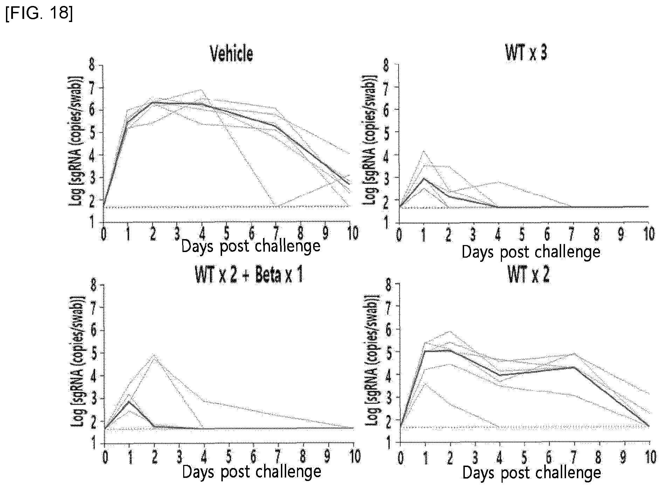

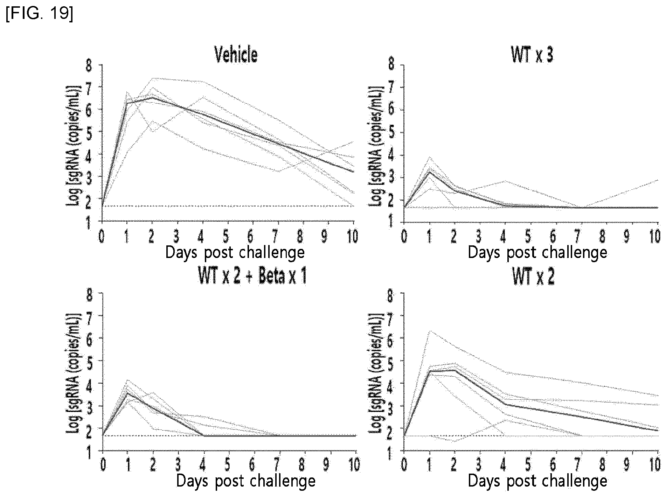

The gene fragments of the subgenomic E gene were first synthesized to prepare a standard. A Taqman custom gene expression assay was designed using the sequence targeting the E gene sgRNA. Primers and probes were identical for both variants. Subsequently, the gene fragments were cloned into the pcDNA3.1+ expression plasmid. The inserted genes were transcribed into RNA *in vitro* using the AmpliCap-Max T7 High Yield Message Maker Kit. Standard log dilutions ranging from 1×10¹⁰ copies to 1×10⁻¹ copies were prepared for RT-PCR analysis. The reaction was performed twice for samples and standards on the QuantStudio 6 and 7 Real-Time PCR Systems with initial denaturation at 95°C for 20 seconds, followed by 45 cycles of denaturation at 95°C for 1 second and annealing at 60°C for 20 seconds. The sub-genomic RNA per mL or per swab was calculated using the standard curve. The sensitivity of the quantitation was 50 copies per mL or per swab. The experimental results are shown in FIGS. 18 and 19.

### Example 5-2. Experimental Results

After the second or third immunization, ELISA was performed to measure the titers of IgG antibodies specific for the wild-type (Wuhan strain), Beta, or Delta RBD in the serum (FIG. 14). The RBD-specific IgG antibodies were induced in all immunized NHPs. High titers were observed in the third vaccination group, while the titers gradually decreased in the group that did not receive the third vaccination.

Similar to the total antibody analysis, high neutralizing antibody titers were induced after the third vaccination, while the titers gradually decreased in the group that did not receive the third vaccination. The PBNA analysis results also indicate that vaccination induced high levels of neutralizing antibodies against the wild-type and variant SARS-CoV-2, and in particular, the booster injection of RBD-NP provided sufficient cross-protection against the wild-type and variants (Beta, Delta, and Omicron) of SARS-CoV-2 (FIG. 15 and FIG. 16).

Similar to the results on the study using mice, total and neutralizing antibody titers against SARS-CoV-2 variants were improved by the third vaccination, suggesting that booster shots can increase protection against multiple variant viruses.

Additionally, the neutralizing antibody titers were compared after the second immunization with the RBD or recombinant spike proteins of the Preparation Example of the present disclosure, and as a result, it was confirmed that the group administered with the RBD of the Preparation Example of the present disclosure had a higher neutralizing antibody formation against the Omicron variant (FIG. 16). This implies that the booster shot vaccination using the composition of the present disclosure provides protection against the Omicron variant.

On day 70 after the first vaccination, PBMC isolated from whole blood were treated with a stimulating antigen to identify T cells secreting IFN-γ using ELISpot analysis. As a result, it was confirmed that the number of T cells specifically reacting to the wild type, Delta, or Beta peptides increased in the vaccination group. Additionally, the IFN-γ secreting T cells significantly increased in the third administration group compared to the second administration group (FIG. 17).

On day 77 after the first vaccination, rhesus macaques were challenged intranasally and intratracheally with the Delta variant strain (B.1.617.2) at a dose of 5 × 10⁵ TCID/head. After 10 days, nasal swabs and bronchoalveolar lavage were collected to identify the virus load using the subgenomic RNA copies. As a result, the viral clearance was specifically accelerated in the third administration group (FIGS. 18 and 19).

### Example 6. Verification of Formation of Cross-Immunity via FRNT Test

This study consisted of five cohorts for evaluating the safety, tolerability, and immunogenicity of GBP510 (referring to the RBD nanoparticle-AS03 formulation prepared in Preparation Example 1 in Example 6) at the first booster vaccination, and two cohorts for evaluating the safety, tolerability, and immunogenicity of GBP510 at the second booster vaccination.

For the first booster vaccination group, it was designed to recruit approximately 550 adults aged 19 years or older (110 per cohort depending on the type of COVID-19 vaccine previously administered) who completed the primary vaccination schedule 12 to 24 weeks ago via the same or cross-vaccination of domestically approved COVID-19 vaccines. Each cohort in the first booster vaccination group was divided into five cohorts (Cohorts 1 to 5) who received the same primary vaccination with ChAdOx1 nCOV-19 (AstraZeneca), BNT162b2 (Pfizer), mRNA-1273 (Moderna), or Ad26.COV2.S (Janssen) or crossed vaccination with ChAdOx1 nCOV-19 (AstraZeneca) & BNT162b2 (Pfizer), depending on the type of primary vaccination.

For the second booster vaccination group, it was designed to recruit approximately 220 adults aged 19 years or older (110 per cohort) who had completed the primary vaccination and the first booster vaccination through the same or cross-vaccination of ChAdOx1 nCOV-19 (AstraZeneca), Ad26.COV2.S (Janssen), NVX-CoV2373 (Novavax), BNT162b2 (Pfizer), and mRNA-1273 (Moderna) at least 16 weeks ago. The recruited subjects were divided into Cohort 6, who received both the primary vaccination and the first booster vaccination with the same or crossed mRNA vaccine (BNT162b2 (Pfizer) or mRNA-1273 (Moderna)), and Cohort 7, who received at least one non-mRNA vaccine (ChAdOx1 nCOV-19 (AstraZeneca), Ad26.COV2.S (Janssen), NVX-CoV2373 (Novavax)).

The enrolled subjects were randomly assigned to the test group or the placebo group in a 10:1 ratio for each cohort and received a single vaccination of GBP510 or placebo. The test group subjects who received GBP510 were monitored for approximately 12 months after vaccination, and the placebo group for approximately 4 weeks in the first and second booster vaccination groups. Immunogenicity blood samples were collected before the GBP510 booster vaccination, and at 2 weeks, 4 weeks, 3 months, 6 months, and 8 months after vaccination for the test group, and for the placebo group, before the placebo vaccination, and at 2 weeks and 4 weeks after vaccination.

Among the 212 subjects in the first booster vaccination group enrolled by the interim analysis, a subgroup of 60 subjects randomly selected from the test group vaccinated with GBP510 were analyzed for cross-reactivity on the specific variants of concern using the focus reduction neutralization test (FRNT). Blood samples collected before and 2 weeks after the GBP510 booster vaccination were analyzed to calculate the pre- and post-vaccination FRNT50 neutralizing antibody titers for the BA.1, BA.5, XBB.1, and BN.1 variants, and the results were compared relative to the Wuhan strain by a foldreduction comparison.

Vero or Vero E6 cells were seeded into each well of a 96-well plate at 70% confluency. The initial dilution concentration of the sample used for analysis was 1/10, and the samples were then diluted by a 2-fold to 4-fold dilution ratio. The virus was diluted to an appropriate concentration of 100 to 600 FFU, mixed with the sample at a 1:1 ratio, and neutralized for 30 minutes to 1 hour and 30 minutes. 20 to 100 µL of the neutralized mixture was added per well where cells were seeded, and infected for 4 to 9 hours in a 5% CO₂ incubator. After infection, the cells were fixed by adding a 10% formalin solution or paraformaldehyde. Thereafter, a permeabilization solution was added to the cells for treatment, washed with PBS, and 100 µL of the SARS-COV-2 antibody was added per well and reacted for 1 hour. After washing again with PBS, 100 µL of a secondary antibody was added per well and reacted for 1 hour, then the cells were washed with PBS, and true blue was added to each well to develop color. After color development, the plates were measured with a foci counting device, and the foci of the measured neutralized samples were compared with the foci of the non-neutralized wells to confirm the dilution ratio at which the foci were reduced by 50%, and the value was calculated as the ND50 value.

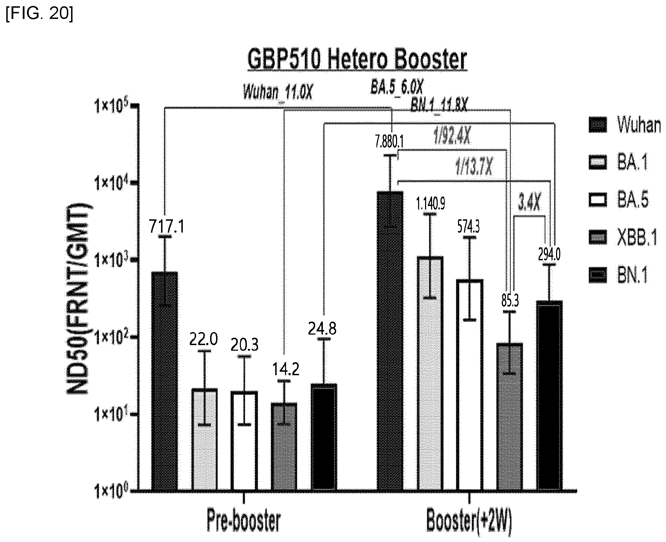

The experimental results are shown in FIG. 20.

The experimental results confirmed that the booster shot of the RBD nanoparticle AS03 formulation could provide protection not only against the wild type Wuhan strain but also against various Omicron subspecies.

From the foregoing, a skilled person in the art to which the present disclosure pertains will be able to understand that the present disclosure may be embodied in other specific forms without modifying the technical concepts or essential characteristics of the present disclosure. In this regard, the exemplary embodiments disclosed herein are only for illustrative purposes and should not be construed as limiting the scope of the present disclosure. On the contrary, the present disclosure is intended to cover not only the exemplary embodiments but also various alternatives, modifications, equivalents, and other embodiments that may be included within the spirit and scope of the present disclosure as defined by the appended claims.

## Claims

1. A booster vaccine composition for inducing or maintaining an immune response against a wild-type and variant SARS-CoV-2 in an administered subject,
wherein the booster vaccine composition comprises:
(i) a receptor binding domain (RBD) nanoparticle, comprising (a) a trimer consisting of three assembled first polypeptide monomers comprising an amino acid sequence of SEQ ID NO: 1 or an amino acid sequence having at least 75% identity thereto; and (b) a pentamer consisting of five assembled second polypeptide monomers comprising an amino acid sequence of SEQ ID NO: 2 or an amino acid sequence having at least 75% identity thereto, and
(ii) AS03,
wherein the subject has been vaccinated at least once with a vaccine composition against SARS-CoV-2 or has a history of infection with SARS-CoV-2.

2. The composition of claim 1, wherein the first polypeptide monomer of (i) (a) of the booster vaccine composition comprises an RBD sequence selected from SEQ ID NO: 21 and SEQ ID NO: 22.

3. The composition of claim 1, wherein the booster vaccine composition provides cross-protection against a wild-type and variant SARS-CoV-2 viruses.

4. The composition of claim 1, wherein the variant SARS-CoV-2 virus is any one or more selected from Alpha, Beta, Delta, Gamma, and Omicron variants.

5. The composition of claim 1, wherein the vaccine composition administered to the subject comprises:
(i) a receptor binding domain (RBD) nanoparticle, comprising (a) a trimer consisting of three assembled first polypeptide monomers comprising an amino acid sequence of SEQ **ID** NO: 1 or an amino acid sequence having at least 75% identity thereto; and (b) a pentamer consisting of five assembled second polypeptide monomers comprising an amino acid sequence of SEQ **ID** NO: 2 or an amino acid sequence having at least 75% identity thereto, and
(ii) AS03.

6. A SARS-CoV-2 vaccine composition for use in a method for inducing or maintaining an immune response against SARS-CoV-2 in a subject, comprising:
a first step of administering a prime vaccine composition against SARS-CoV-2; and a second step of administering a booster vaccine composition against SARS-CoV-2 at least 14 days after the first step,
wherein the SARS-CoV-2 vaccine composition of the second step comprises:
(i) a receptor binding domain (RBD) nanoparticle, comprising (a) a trimer consisting of three assembled first polypeptide monomers comprising an amino acid sequence of SEQ **ID** NO: 1 or an amino acid sequence having at least 75% identity thereto; and (b) a pentamer consisting of five assembled second polypeptide monomers comprising an amino acid sequence of SEQ **ID** NO: 2 or an amino acid sequence having at least 75% identity thereto, and
(ii) AS03.

7. The composition of claim 6, wherein the interval between the first and second steps is 28 days or more.

8. The composition of claim 6, wherein the prime vaccine composition of the first step comprises a viral vector, nucleic acid or synthetic antigen as an active ingredient.

9. The composition of claim 6, wherein the prime vaccine composition of the first step comprises:
(i) a receptor binding domain (RBD) nanoparticle, comprising (a) trimer consisting of three assembled first polypeptide monomers comprising an amino acid sequence of SEQ ID NO: 1 or an amino acid sequence having at least 75% identity thereto; and (b) a pentamer consisting of five assembled second polypeptide monomers comprising an amino acid sequence of SEQ ID NO: 2 or an amino acid sequence having at least 75% identity thereto, and
(ii) AS03.

10. The composition of claim 6, wherein the SARS-CoV-2 vaccine composition of the second step comprises the first polypeptide monomer of (i) (a) of the SARS-CoV-2 vaccine composition in the second step and comprises an RBD sequence selected from SEQ ID NO: 21 and SEQ ID NO: 22.

11. The composition of claim 6, wherein the SARS-CoV-2 vaccine composition of the second step is a composition, in which a composition, which comprises an RBD nanoparticle consisting of a first polypeptide monomer comprising an RBD sequence of SEQ ID NO: 21; and a second polypeptide monomer comprising an amino acid sequence of SEQ ID NO: 2, and AS03; and
a composition, which comprises an RBD nanoparticle consisting of a first polypeptide monomer comprising an RBD sequence of SEQ ID NO: 22; and a second polypeptide monomer comprising an amino acid sequence of SEQ ID NO: 2, and AS03, are mixed.

12. The composition of claim 6, wherein the SARS-CoV-2 vaccine composition of the first step comprises an RBD sequence of SEQ ID NO: 21.

13. The composition of claim 6, wherein the composition provides cross-protection against a wild-type and variant SARS-CoV-2 viruses.

14. The composition of claim 6, wherein the variant SARS-CoV-2 virus is any one or more selected from Alpha, Beta, Delta, Gamma, and Omicron variants.

15. The composition of claim 6, wherein vaccine composition comprises (i) and (ii) in a ratio of 1:1.

16. The composition of claim 6, wherein the RBD nanoparticles contained in the vaccine composition is 5 µg to 25 µg per single dose.

17. The composition of claim 6, wherein the vaccine composition is administered by intramuscular injection.

18. The composition of claim 6, wherein the composition is provided in the form of a pharmaceutical package or kit.

19. The composition of claim 18, comprising a means for administering the composition to a patient.
